# EUROPEAN PATENT SPECIFICATION

(11) **EP 2 692 724 B1**
(45) Date of publication and mention of the grant of the patent: **16.03.2016**
(21) Application number: 11862562.3
(22) Date of filing: 22.12.2011
(51) Int. Cl.: C07D 309/40, A61K 31/351, A61P 39/04, A61P 7/06, A61P 25/28, A61P 35/00, A61P 25/00

(54) **CHIRAL 3-HYDROXYPYRID-4-ONE DERIVATIVE, AND SYNTHESIS AND USE THEREOF**
CHIRALES 3-HYDROXYPYRID-4-ON-DERIVAT SOWIE SEINE SYNTHESE UND VERWENDUNG
DÉRIVÉ CHIRAL DE 3-HYDROXYPYRID-4-ONE ET SYNTHÈSE ET UTILISATION DE CELUI-CI

(30) Priority: 31.03.2011 CN 201110080525
(43) Date of publication of application: 05.02.2014
(73) Proprietor: Zhejiang University, Xihu District Hangzhou Zhejiang 310027 (CN)
(72) Inventor: HIDER, Robert, St Osyth Essex CO 16 8JL (GB); LIU, Zudong, Hangzhou Zhejiang 310012 (CN); YU, Yongping, Hangzhou Zhejiang 310027 (CN); LI, Zhi, Hangzhou Zhejiang 310027 (CN)
(74) Representative: Decamps, Alain René François
(86) International application number: PCT/CN2011/084489
(87) International publication number: WO 2012/129942

(56) References cited:
- EP-A2- 0 120 669
- EP-A2- 0 120 669
- WO-A1-94/03169
- WO-A1-94/03169
- CN-A- 102 190 644
- Anonymous: "IUPAC Gold Book - chiral", , 1 January 1996 (1996-01-01), XP055127889, Retrieved from the Internet: URL:http://goldbook.iupac.org/C01057.html [retrieved on 2014-07-09]
- DATABASE REGISTRY [Online] CHEMICAL ABSTRACTS SERVICE, COLUMBUS, OHIO, US; 9 March 2011 (2011-03-09), "4(1H)-Pyridinone, 2-ethyl-3-hydroxyl-1-[1-(hydroxymethyl)pro pyl]-", XP002727055, Database accession no. 1267228-85-2
- JOHN JOSEF MOLENDA ET AL: "Mobilization of Iron by Chiral and Achiral Anionic 3-Hydroxypyrid-4-ones", JOURNAL OF MEDICINAL CHEMISTRY, vol. 37, no. 25, 1 December 1994 (1994-12-01), pages 4363-4370, XP055127833, ISSN: 0022-2623, DOI: 10.1021/jm00051a014
- RAI, BIJAYA L. ET AL.: 'Synthesis, Physicochemical Properties, and Evaluation of N-Substituted-2-alkyl-3-hydroxy-4(lH)-pyrid inones' JOURNAL OF MEDICINAL CHEMISTRY vol. 41, no. 18, 1998, pages 3347 - 3359, XP002064973

## Description

### Technical Field

The present invention relates to the field of drug synthesis and biological effect, purpose of which is to develop brand new Chiral 3-hydroxypyridin-4-one Fe(III) chelators. This series of compounds are designed to treat toxicity symptoms caused by excessive accumulation of iron in vivo, such as thalassemia and other diseases dependent on long-term transfusion, as well as the injury due to excessive accumulation of iron in local tissues, which has special medicinal value.

### Background Art

Many diseases in humans and animals are caused by excessive accumulated metals, such as iron. Among such diseases, excess iron is accumulated in various tissues, which is called iron overload disorders, formerly known as siderosis Haemorrhagic. Excess iron has the following sources: 1) long-term blood transfusion; 2) the gastrointestinal system absorbing excess iron, because stimulated by diseases such as anemia. It is necessary to repeat transfusion for some patients with severe anemia, for example, β-thalassemia, as well as other anemia requiring transfusion therapy. Excessive iron absorption from the gastrointestinal tract usually occurs in hemochromatosis patients and in anemia patients who do not require blood transfusion, such as thalassemia intermedia. If iron overload disease is not treated, it will result in severe tissue damage, especially the liver, heart and endocrine organs, and ultimately lead to death. Iron chelators can remove and clear excess iron from such organs, relieve symptoms and reduce the corresponding mortality.

Desferrioxamine (DFO) is an effective iron chelator for a long time. However, in the treatment of the diseases mentioned above, the biggest disadvantage regarding DFO and its salts is its poor oral absorption capability. So, administration is achieved with a slow injection method (8∼12h/day), patients need to wear a portable drug delivery device during treatment, such as mounting the syringe on a mechanical pressing device. This method is inconvenient, and also expensive, which largely limits the utilization of DFO, especially for thalassemia-prone areas, such as Mediterranean, Middle East, and India &South East Asia, it plays no role in treatment of malaria in world-wide and sickle cell anemia in some African countries, which is a very serious problem to the populations there.

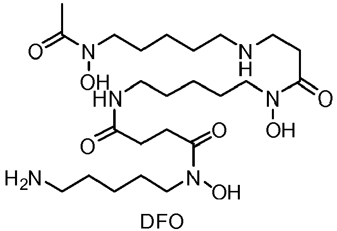

UK Patent No. 2, 13, 807, US Patent No. 4, 585, 780 and other scientific research have reported the treatment of iron overload symptoms by using 3-hydroxypyridin-4-one derivatives, especially in some pathological symptoms, such as thalassemia, sickle cell anemia, aplastic anemia in children, and idiopathic hemochromatosis, usually, treatment of the first three diseases includes frequent regular blood transfusion. 3-Hydroxypyridin-4-one derivatives, especially CP20 (commercial named Ferriprox) is employed to treat systemic iron overload disorders, and also to treat certain diseases associated with local iron overload distribution, although such patients do not show symptoms of systemic iron overload, i.e. inhibition free radical mediated reactions caused by excess iron ions in certain neurodegenerative diseases and cancer diseases. A serious limitation of CP20 is that the hydroxyl group at 3' position is vulnerable to glycosylation, which reduces the half-life of this compound (approximately 2∼3 h). So it requires a high dosage, which is associated with obvious side effects.

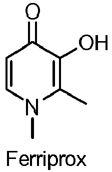

EP0120669 discloses compounds with a 3-hydroxypyrid-4-one in which the H attached to the N atom is substituted by an aliphatic acyl group, or an aliphatic hydrocarbon group, these groups can be further substituted, but not by aromatic groups and their use against illnesses related to iron overload. Molenda et al. disclose in Journal of Medicinal Chemistry 1994, 37, pages 4363-4370 chiral 3-hydroxy-pyridin-4-one compound 6 as enhancing iron excretion.

US Patent No. 6, 465, 604 described a series of 3,5-diphenyl-1,2,4-triazole compounds, wherein including Exjade (commercial name), which has strong affinity to Fe(III), However, its active groups contain two negatively charged oxygen ions and a carboxyl group; it is a tridentate ligand while chelating Fe(III), which forms a Fe-L₂ type complex, possessing three unit negative charges itself, that is bad for their discharge from cells/tissues. Moreover, one of the active groups is a nitrogen atom with a lone pair of electrons, Exjade may have a negative effect on the balance of Zn(II) in vivo, at the same time because it has two phenolic hydroxyl groups in different positions (forming intramolecular hydrogen bonds structure similar to cis/trans isomerization), it can be complexed to several zinc ions to form high molecular weight polymers complexes, which is not conducive to its discharge from the cells either.

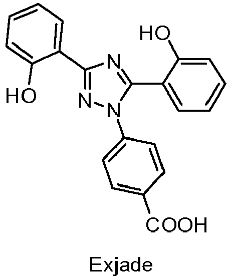

Absorption, distribution, metabolism and excretion of chiral medicines are largely related to the 3D structures of their chiral centers. For drug absorption, chiral compounds entering cells via active transport mechanism are usually carried by special transport proteins, their recognition of enantiomers can be different, resulting in different absorption of enantiomers. For drug distribution, the binding effects of plasma protein and tissues are also somewhat stereoselective, leading to different in vivo distribution of enantiomers; for stereoselective of drug metabolism refers to when the substrate is biotransformated, the pathway and speed of enantiomer metabolism by biological systems can be different. One enantiomer may show ascendant metabolism, and therefore it is of great significance to the indicators including drug transformation and in vivo half-life. Glomerular filtration, tubular secretion and reabsorption of chiral drugs to clear the chiral drugs, having stereoselectivity, while the glomerular filtration rate is closely related to drug's selectivity to binding plasma protein, so discharge style of enantiomers (urine / feces percentage) and the rate is also different.

Therefore, the qualitative difference of the interactions of a pair of enantiomers with various binding sites may exist or not, and the quantitive difference may exist (strong or weak), which results in the different activities between enantiomers. Thus the selection of optical enantiomers for medical use, requires a comprehensive study of metabolic activity, toxicology and pharmacokinetic properties etc. Thus the chiral nature of the 3-hydroxypyridin-4-one derivatives described in this patent has an important role on in vivo iron chelation.

The effectiveness of many oral 3-hydroxy-4-one derivatives drugs are subject to metabolic reaction of the 3-hydroxy moiety, which may be quickly glycosylated (see **Reaction I**). The hydroxypyridone after glycosylation loses the ability to chelate Fe(III). We can effectively inhibit glycosylation reaction by introducing hydroxyl groups to alkyl substituted residues on the pyridine ring. In addition, the partition coefficient of 3-hydroxy-pyridin-4-one derivatives has a great impact on the in vivo distribution and toxic effects. We have introduced various alkyl groups to the chiral point of the compound, in order to modify their lipophilicity, i.e. a phenyl group connected to the chiral point in compound **IV-b** while in **IV-a** it is a methyl group, and thus compound **IV-b** is relatively more lipophilic, and easier to penetrate through cell membranes of various tissues and critical barriers such as the blood-brain barrier and the placental barrier, thus affecting its in vivo distribution. Thus increase of hydroxyl groups can affect the intestinal absorption capacity, by introducing a large alkyl group, intestinal absorption of 3-hydroxy-pyridin-4-one derivatives can be enhanced.

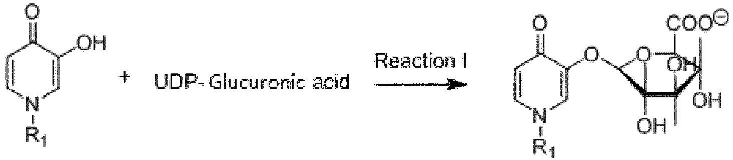

### Reaction I

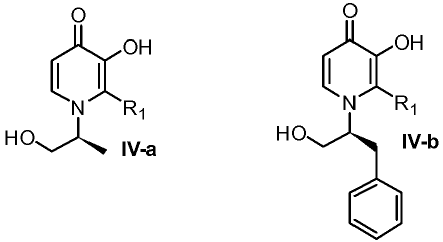

### Summary of the Invention

The present invention is directed to provide a series of chiral 3-hydroxypyridin-4-one derivatives and their salts, having the following general formula **I:**

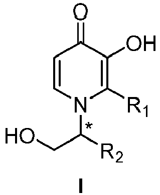

Wherein, the carbon marked with asterisk has chirality.

R₁ and R₂ are same or different, and independently represent alkyl, hydroxy alkyl, mercapto alkyl, haloalkyl, alkoxy, alkylthiol, haloalkoxy, haloalkylthiol, carboxyl alkyl, alkoxyacyl-alkyl, R₃R₄N-C(O)-alkyl, heterocycloalkyl, unsubstituted aryl, aroyl, aralkyl, unsubstituted heterocyclic aryl or heterocyclic aralkyl.

Said alkyl represents methyl, ethyl, n-propyl, isopropyl, n-butyl, isobutyl, n-pentyl, n-hexyl or n-heptyl, usually represents methyl, ethyl and n-propyl.

Said hydroxy alkyl is an alkyl substituted by hydroxy, wherenin hydroxyl is at the terminal of alkyl, such as 3-hydroxypropyl; or hydroxyl is in the middle of alkyl, such as 2-hydroxypropyl, 1-hydroxypropyl or 1-hydroxy-2-methylpropyl.

Said mercapto alkyl refers to a group in which oxygen atom in hydroxy alkyl is substituted by a sulfur atom. The halogen alkyl refers to alkyl substituted by a halogen atom, usually chlorine or fluorine, also can be substituted by trifluoro or trichloro halogen substitution site is at the terminal or in the middle of alkyl.

The alkoxy group refers to a group formed by alkyl and oxygen atom which is covalently linked to the substitution site via an ether linkage, such as methoxyl, oxethyl, n-propoxyl, isopropoxy, n-butoxy, isobutoxy, sec-butoxy, tert-butoxy, n-pentyloxy, isopentyloxy, usually methoxy and oxethyl.

The alkylthiol group is a group in which the oxygen atom in alkoxy is substituted by sulfur atom.

The haloalkoxy refers to an alkoxy substituted by halogen , generally chlorine or fluorine, may also be substituted by trichloro or trifloro.

The haloalkylthiol refers to a group in which oxygen atom in haloalkoxy is substituted by sulfur atom.

The carboxyl alkyl refers to an alkyl substituted by carboxyl including, a single carboxyl substitution, or multi-carboxyl substitution, the substitution site is at the terminal or in the middle of the alkyl.

The alkoxyacyl refers to "alkyl-O-C(O)-", such as methoxycarbonyl, ethoxycarbonyl, n-propoxycarbonyl, isopropoxycarbonyl, isobutoxycarbonyl, sec-butoxycarbonyl, tert-butoxycarbonyl, n-pentyloxycarbonyl, isopentyloxycarbonyl, generally refers to methoxycarbonyl and ethoxycarbonyl.

R₃ and R₄ in R₃R₄N-C(O)-alkyl are same or different, which can be hydrogen, alkyl, hydroxy alkyl, alkoxy-alkyl, hydroxy alkoxy-alkyl, amino-alkyl, N-alkylamino-alkyl, N,N-dialkylamino-alkyl, N-hydroxyalkylamino-alkyl, N,N-dihydroxyalkylamino-alkyl, or a nitrogen-containing alicyclic ring formed by connecting two terminals of alkyl with a nitrogen atom.

The heterocyclic alkyl refers to a ring structure containing 3-8 atoms (mainly 5-7 atoms), wherein there is at least one heteroatom, generally oxygen, nitrogen or sulfur atom, i.e. a nitrogen-containing heterocycle refers to a ring structure containing 3-8 atoms (mainly 5-7 atoms), with at least one nitrogen atom; nitrogen-containing heterocycle can also have other hetero atom, such as oxygen, nitrogen or sulfur atom, for example, piperidyl, piperazinyl, morpholinyl or pyrrolidinyl etc.

The aryl refers to an unsubstituted phenyl.

The aroyl refers to "aryl-C(O)-", such as benzoyl, toluoyl, naphthoyl or p-methoxy benzoyl.

The arakyl refers to a strand of alkyl that contains at least one hydrogen atom, typically the hydrogen attached to the terminal carbon is substituted by an aryl , such as benzyl, *p*-chlorobenzyl, *o*-fluorobenzyl, phenethyl, *p*-methylbenzyl, *p*-dimethylamino benzyl, *p*-diethylamino benzyl, *p*-cyanobenzyl or *p*-pyrrolidinyl benzyl.

The heterocyclic aryl itself refers to an aromatic ring containing a hetero atom, or component of other groups, such as aromatic heterocycle-acyl; aromatic heterocycle refers to an aromatic ring containing 3-7 atoms (mainly 5-7 atoms), wherein there is at least one heteroatom, such as pyrrolidinyl, imidazolyl, triazolyl, tetrazolyl, oxygen nitrogen cyclopentadienyl , thiazolyl, furanyl, thiophenyl, pyridyl, pyrazinyl, thiazinyl, pyranyl, or pyrimidyl. The aromatic heterocycle is unsubstituted.

The heterocyclic aralkyl refers to a strand of alkyl with at least one hydrogen atom, generally means the hydrogen atom attached to the terminal carbon atom is substituted by an aromatic heterocycle. The aromatic heterocycle has one or more substituents, usually one or two, such as alkyl, halogen, trifluoromethyl, carboxyl, alkoxyacyl, N-alkylamino, N,N-dialkylamino or pyrrolidinyl.

The present invention also contains corresponding salts formed by compound **of formula I** and an acid. The acid may be a strong inorganic acid, typically a mineral acid, such as sulphonic acid, phosphoric acid or hydrocholic acid. The acid may be a strong organic acid as well, generally an alkyl carboxylic acid, specifically acetic acid; or the acid can be a saturated or unsaturated dicarboxylic acid , such as tartaric acid or citric acid. The acid may also be an alkyl-substituted or unsubstituted phenylsulfonic acids , specifically methyl or p-toluenesulfonic acid.

Specially, when R₁ in formula **I** is methyl, and compound is shown in formula **II**: 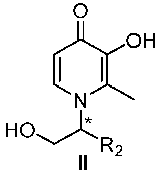 wherein the carbon atom marked with "*" is chiral, R₂ is defined as that in formula **I** Preferred compound of formula **II** is selected from:
II-a: (*S*)-3-Hydroxy-1-(1-hydroxy propyl-2-)-2-methyl pyridine-4(1*H*)-one;
II-b: (*S*)-3-Hydroxy-1-(1-hydroxy-3-methyl butyl-2-)-2-methyl pyridine-4(1*H*)-one;
II-c: (*R*)-3-Hydroxy-1-(1-hydroxy-3-phenyl propyl-2-)-2-methyl pyridine-4(1*H*)-one;
II-d: (*S*)-3-Hydroxy-1-(1-hydroxy-3-methyl pentyl-2-)-2-methyl pyridine-4(1*H*)-one.

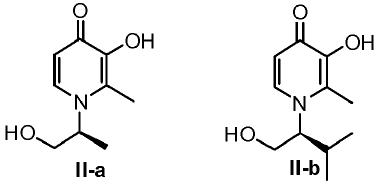

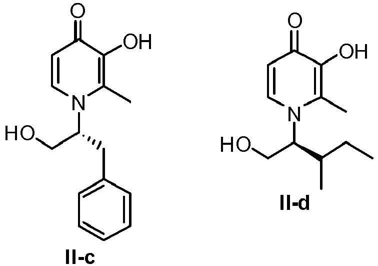

When R₁ in formula **I** represents ethyl, and compound is shown in formula **III:** 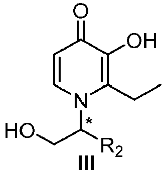 wherein the carbon atom marked with "*" is chiral, R₂ is defined as that in formula I; Preferred compound of formula III is selected from:
III-a: (*S*)-3-Hydroxy-1-(1-hydroxy-3-methyl butyl-2-)-2-ethyl pyridine-4(1*H*)-one;
III-b: (*S*)-3-Hydroxy-1-(1-hydroxypropyl-2-)-2-ethylpyridine-4(1*H*)-one;
III-c: (*R*)-3-Hydroxy-1-(1-hydroxy-3-phenyl propyl-2-)-2-ethyl pyridine-4(1*H*)-one.

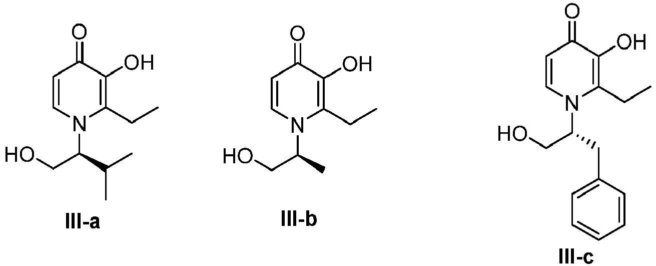

On the other hand, the present invention is directed to provide the synthetic method of the described series of chiral 3-hydroxypyridin-4-one derivatives and their salts: reacting methyl (or ethyl) maltol (compound **V)** with benzyl chloride (compound **VI**) to form a 3'-benzyl protected maltol (compound **VII),** then reacting compound **VII** with different chiral amino alcohols (compound **VIII)** to get chiral 3-benzyloxypyridin-4-ones (compound **IX),** finally, deprotect compound **IX** in the presence of Pd/C as hydrogenation catalyst to form compound of formula **I,** the reaction scheme is as follows: 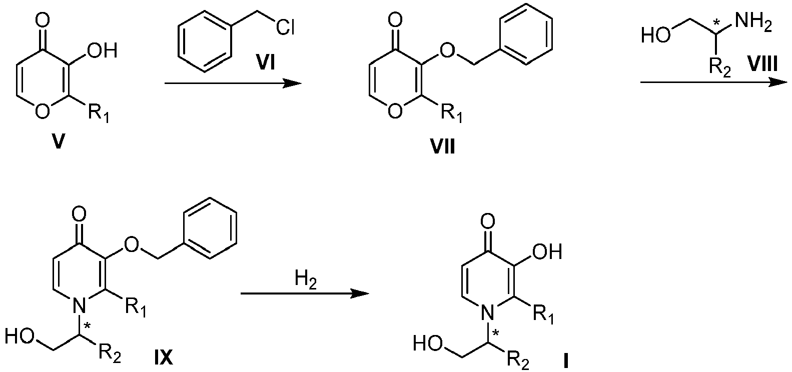 and reacting compound of formula I with an inorganic or organic acid to form corresponding salts, and the reaction scheme is as follows:

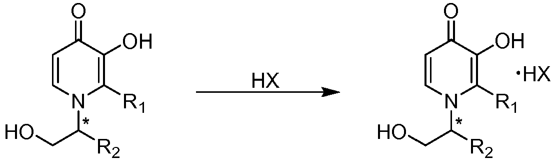

HX is an inorganic or organic acid.

This patent also provides the use of the described series of chiral 3-hydroxypyridin-4-one derivatives and their salts for the production of a medicament for anti-iron overdose. As showed in the tests: the series of chiral 3-hydroxypyridin-4-one derivatives have the ability of chelating and removing overloaded metal ions, especially iron ions in human and animals. They have good oral activity and substantial iron removal activity.

This patent describes the formulations of the prepared compounds, including liquid form and solid form, such as granule, tablet or capsule. The mode of administration of the described compounds is oral or injection.

Advantages of this patent are: optimal designing of 3-hydroxypyridin-4-one derivatives, adding chiral groups to enhance their bioactivity and reduce toxicity. Chiral 3-hydroxypyridin-4-one derivatives described have good bioactivity of chelating and removing overloaded metal ions, especially iron accumulated in human and animals. They also have good oral activity and eminent iron removal activity. the vivo test of rats shows that the iron removal effect is superior to the market available CP20, thus a few of several preferred compounds have high potential to become new generation medicines for treatment of iron overload, specifically thalassemia and other diseases which require long term massive blood transfusion, and tissue damage caused by local iron accumulation, such as iron-mediated tissue lesions in neurodegenerative diseases and onco-diseases, for example, heart, liver etc.

### Detailed Description:

Following will further illustrated the invention with specific embodiments.

### Example Part (One) synthesis of chiral 3-hydroxypyridin-4-one derivatives

### Example 1. 3-benzyloxy-2-methyl-4H-pyran-4-one

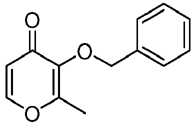

122.3 g NaOH was dissolved into 278 mL water, then mixed into solution of 390 g maltol and 1160 mL ethanol, and 422 g benzyl chloride was then added under stirring. Keep stirring at 60°C for 3.5 h after fully mixing. After cooling the reaction system, filter it to remove solid impurities, wash the residues with ethanol, which was then mixed with filtrate and spin-dried. The remaining substances were dissolved by methylene chloride (DCM), then washed with 5% NaOH, finally washed with suitable amount of water. The organic layer was dried by anhydrous Na₂SO_{4,} then spin-dried the solvent the yellow oily liquid was obtained. After cooling and recrystallization in ether to get crystals, the yield is 78%, melting point is 56∼57°C.

### Example 2. 3-benzyloxy-2-ethanol-4H-pyran-4-one

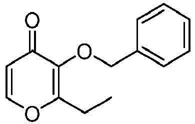

122.3 g NaOH was dissolved into 278 mL water, then mixed into solution of 390 g ethyl maltol and 1160 mL ethanol, and 422 g benzyl chloride was then added under stirring. Keep stirring at 60°C for 3.5 h after fully mixing. After cooling the reaction system and filtration to remove solid impurities, wash the residues with ethanol, which was then mixed with filtrate and spin-dried. The resulting chemicals were dissolved by DCM, then washed with 5% NaOH, finally washed with suitable amount of water. The organic layer was dried by anhydrous Na₂SO₄, spin-dried the solvent to get yellow oily liquid. After cooling and recrystallization in ether, the yield is 83%, melting point is 84∼85°C.
¹HNMR (CDCl₃) δ 1.0 (t, 3H), 2.55 (q, 2H), 5.13 (s, 2H), 6.3 (d, 1H), 7.35 (s, 5H), 7.6 (d, 1H).

### Example 3. Also disclosed in the present application: (S)-3-hydroxy-1-(1-hydroxy propyl-2-)-2-methyl pyridine-4(1H)-one hydrochloride, CN306.

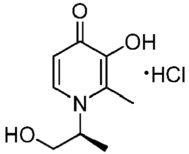

60 g 3-phenyloxy-2-methyl-4H-pyran-one (Example 1) was dissolved into 150 mL n-butanol, then 41.6 g L-alaninol was added. After thoroughly mixing, the solution was refluxed at 118°C for 36 h. After cooling and filtering, the residue was washed with acetic ester, to obtain white solid, which was then dissolved into 150 mL ethanol and 15 mL water, then introducing H and get rid of benzyl under hydrogenation with 5% Pd/C as catalyst. The solvent was removed under rotary evaporation, the remaining solid was recrystallized with methanol and diethylether. The crude product was then treated with HCl methanol dissolved to get hydrochloride. After recrystallization, 44.30 g white solid was obtained with the yield of 73.8%. specific rotation [α]¹⁸_{D}= +11.57, ¹HNMR (DMSO-*d₆*) δ 1.43 (d, J=7, 3H), 2.59 (s, 3H), 3.67 (dd, J₁=8, J₂=12.5, 1H), 3.74 (dd, J₁=4, J₂=12, 1H), 4.87 (m, 1H), 7.30 (m, 1H), 8.32 (d, J=7.5, 1H).

### Example 4. Also disclosed in the present application: (S)-3-Hydroxy-1-(1-hydroxy-3-methyl butyl-2-)-2-methyl pyridine-4(1H)-one, Number : CN116.

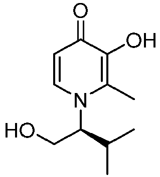

60 g 3-phenyloxy-2-methyl-4H-pyran-one **(Example 1)** was dissolved in 150 mL n-butanol, then 56.1 g L-Valinol was added. After thoroughly mixing, the solution was refluxed at 118°C for 36 h. After cooling and filtration, the product was isolated by silica gel column chromatography with eluent ethanol : ethyl acetate=1:40, after finishing eluting, yellow solid product was obtained after rotary evaporation, which was then dissolved into 150 mL ethanol and 15 mL water, then it was hydrogenated and debenzylated with 5% Pd/C as catalyst, the solvent was removed under rotary evaporation, and the remaining solid was recrystallized with methanol and ether to obtain 17.91 g white solid, the yield is 30.06%, the free alkali's ¹HNMR (DMSO-*d₆*) δ 0.64 (d, J=6.5, 3H), 1.02 (d, J=6.5, 3H), 2.08 (m, 1H), 2.29 (s, 1H), 3.69 (dd, J₁=11.5, J₂=8), 3.78 (dd, J₁=11.5, J₂=3.5), 3.88 (m, 1H), 6.18 (d, J=7.5, 1H), 7.64 (d, J=7.5, 1H).

### Example 5. Also disclosed in the present application: (S)-3-Hydroxy-1-(1-hydroxy-3-methyl butyl-2-)-2-methyl pyridine-4(1H)-one hydrochloride, Number: CN316.

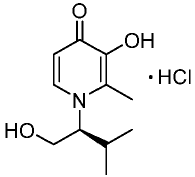

Treated 16 g (*S*)-3-Hydroxy-1-(1-hydroxy-3-methyl butyl-2-)2-methyl pyridin-4(1*H*)-one **(Example 4)** with HCl methanol solution to generate hydrochloride. After recrystallization, 14.93 g white solid was obtained with the yield of 79.6%. specific rotation [α]¹⁸_{D}= -45.48, ¹HNMR (DMSO-*d₆*): δ 0.64 (d, J=6.5, 3H), 1.09 (d, J=6.5, 3H), 2.23(m, 1H), 2.57 (s, 3H), 3.77 (dd, J₁=12, J₂=8.5, 1H), 3.89 (dd, J₁=12, J₂=3.5, 1H), 4.41 (m, 1H), 7.30 (m, 1H), 8.32 (m, 1H).

### Example 6. (S)-3-Hydroxy-1-(1-hydroxy-3-methyl butyl-2-)-2-methyl pyridine-4(1H)-one citrate.

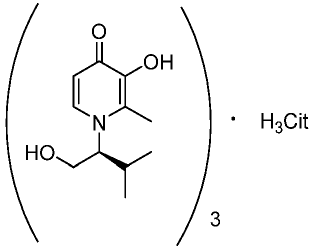

Treated 1 g (*S*)-3-Hydroxy-1-(1-hydroxy-3-methyl butyl-2-)2-methyl pyridin-4(1*H*)-one **(Example 4)** with H₃Cit methanol solution to generate citrate. After recrystallization, 1.08 g white solid was obtained with the yield of 83.3%. ¹HNMR (DMSO-*d₆*): δ 0.6 (d, J=6.5, 9H), 1.11 (d, J=6.5, 9H), 2.27 (m, 3H), 2.59 (s, 4H), 2.64 (s, 9H), 3.61 (br, 1H), 3.88 (dd, J₁=12.5, J₂=8, 3H), 4.04 (dd, J₁=12.5, J₂=4.5, 3H), 4.58 (m, 3H), 7.74 (m, 3H), 8.90 (m, 3H).

### Example 7. (R)-3-Hydroxy-1-(1-hydroxy-3-benzyl propyl-2-)2-methyl pyridine-4(1H)-one, Number: CN128.

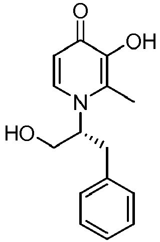

60 g 3-phenyloxy-2-methyl-4H-pyran-one(**Example 1)** was dissolved in 150 mL n-butanol, then 83.7 g D-phenylalaninol was added in. After thoroughly mixing, the solution was refluxed at 118°C for 36 h. After cooling and filtration, products were purified by silica gel column chromatography with Eluent ethanol: acetic ester=1:40. After Elution, light brown solid was obtained after rotary evaporation, which was then dissolved into 150 mL ethanol and 15 mL water, then it was hydrogenated and debenzylated with 5% Pd/C as catalyst, the solvent was removed under rotary evaporation, the remaining solid was recrystallized with methanol and ether, leading to 25.25 g light yellow solid. The yield was 35.1%. The free alkali's ¹HNMR (DMSO-*d₆*): δ 2.00 (s, 3H), 2.98 (dd, J₁=14, J₂=5.5, 1H), 3.11 (dd, J₁=14, J₂=5, 1H), 3.73 (m, 2H), 4.54 (m, 1H), 6.21 (d, J=7, 1H), 7.17 (m, 5H), 7.87 (d, J=7.5, 1H).

### Example 8. (R)-3-Hydroxy-1-(1-hydroxy-3-phenyl propyl-2-)-2-methyl pyridine-4(1H)-one hydrochloride, Number: CN328.

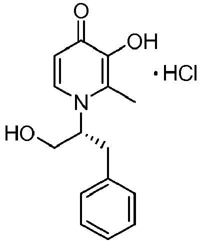

Treated (*R*)-3-Hydroxy-1-(1-hydroxy-3-phenyl propyl-2-)-2-methyl pyridine-4(1*H*)-one **(Example 7)** with HCl methanol solution to generate hydrochloride. Then recrystallization resulted in 18.26 g white solid. The yield was 63.4%. specific rotation [α]¹⁸_{D}= +176.26, ¹HNMR (DMSO-*d₆*): δ 2.30 (s, 3H), 3.13 (dd, J₁=14, J₂=5.5, 1H), 3.28 (dd, J₁=14, J₂=5.5, 1H), 3.87 (m, 2H), 5.08 (m, 1H), 7.20 (m, 5H), 7.48 (m, 1H), 8.62 (m, 1H).

### Example 9. Also disclosed in the present application: 3-Hydroxy-1-((2S)-1-hydroxy-3-methyl pentyl-2-)-2-methyl pyridine-4(1H)-one, Number: CN 136.

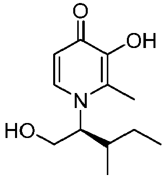

60 g 3-phenyloxy-2-methyl-4H-pyran-4-one **(Example 1)** was dissolved in 150 mL n-butanol, then 64.9g L-isoleucinol was added in. After thoroughly mixing, the solution was refluxed at 118°C for 36 h. After cooling and filtration, products were separated by silica gel column chromatography with eluent ethanol: ethyl acetate= 1:40, light yellow solid product was obtained after rotary evaporation; which was then dissolved into 150 mL ethanol and 15 mL water, then it was hydrogenated and debenzylated with 5% Pd/C as catalyst, the solvent was removed under rotary evaporation, the remaining solid was recrystallized with methanol and diethyl ether, leading to 31.5 g white solid,the yield was 50.4%. The free alkali's ¹HNMR (DMSO-*d₆*): δ 0.75 (t, J=7, 3H), 0.97 (m, 5H), 1.87 (m, 1H), 2.27 (s, 3H), 3.70 (dd, J₁=12, J₂=8, 1H), 3.78 (dd, J₁=12, J₂=3, 1H), 3.93 (m, 1H), 6.17 (d, J=7.5, 1H), 7.64 (d, J=7.5, 1H).

### Example 10. Also disclosed in the present application: 3-Hydroxy-1-((2S)-1-hydroxy-3-methyl pentyl-2-)-2-methyl pyridine-4(1H)-one hydrochloride, Number: CN336.

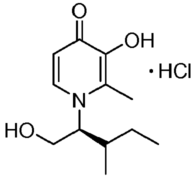

Treated 3-Hydroxy-1-((2*S*)-1-hydroxy-3-methyl pentyl-2-)-2-methyl pyridine-4(1*H*)-one **(Example 9)** with HCl methanol solution to get hydrochloride. The following recrystalization resulted in 25.07 g white solid, the yield was 68.5%. specific rotation [α]¹⁸_{D}= -30.36, ¹HNMR (DMSO-*d₆*) : δ 0.75 (t, J=7.5, 3H), 0.93 (m, 2H), 1.03 (d, J=6.5, 3H), 2.03 (m, 1H), 2.57 (s, 3H), 3.79 (dd, J₁=12, J₂=8.5, 1H), 3.90 (dd, J₁=12, J₂=3.5, 1H), 4.49 (m, 1H), 7.47 (m, 1H), 8.38 (m, 1H).

### Example 11. Also disclosed in the present application: (S)-3-Hydroxy-1-(1-hydroxy-3-methyl butyl-2-)-2-ethyl pyridine-4(1H)-one, Number: CN216.

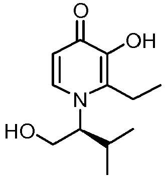

63.8 g 3-benzyloxy-2-ethanol-4H-pyran-4-one **(Example 2**) was dissolved in 150 mL n-butanol, then 56.1 g L-valinol was added in. After thoroughly mixing, the solution was refluxed at 118°C for 36 h. After cooling and filtration, products were purified by silica gel column chromatography with eluent ethanol: ethyl acetate= 1:40, light yellow solid product was obtained after rotary evaporation, which was then dissolved into 150 mL ethanol and 15 mL water, then it was hydrogenated and debenzylated with 5% Pd/C as catalyst. The solvent was removed under rotary evaporation, the residue was recrystallized with methanol and ether, leading to 20.56 g white solid. The yield was 33.1%. The free alkali's ¹HNMR (DMSO-*d₆*): δ 0.65 (d, J=6.5, 3H), 1.04 (d, J=6, 3H), 1.11 (t, J=7.5, 3H), 2.10 (m, 1H), 2.71 (dq, J₁=15, J₂=7.5, 1H), 2.81 (dq, J₁=15, J₂=7.5, 1H), 3.75 (m, 2H), 3.84 (m, 1H), 6.17 (d, J=7.5, 1H), 7.63 (d, J=7.5, 1H).

### Example 12. Also disclosed in the present application: (S)-3-Hydroxy-1-(1-hydroxy-3-methyl butyl-2-)-2-ethyl pyridine-4(1H)-one hydrochloride, Number: CN416.

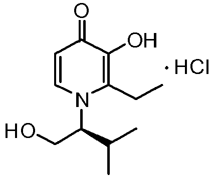

Treated (*S*)-3-Hydroxy-1-(1-hydroxy-3-methyl butyl-2-)-2-ethyl pyridine-4(1*H*)-one **(Example 11)** with HCl methanol solution to obtain hydrochloride. The recrystalization later resulted in 19.85 g white solid, the yield was 82.7%, specific rotation [α]¹⁸_{D}= -42.31, ¹HNMR (DMSO-*d₆*): δ 0.65 (d, J=7, 3H), 1.04 (d, J=6, 3H), 1.11 (t, J=7.5, 3H), 2.11 (m, 1H), 2.72 (dq, J1=15, J2=7.5, 1H), 2.83 (dq, J1=15, J2=7.5, 1H), 3.76 (m, 2H), 3.85 (m, 1H), 6.18 (d, J=7.5, 1H), 7.64 (d, J=7.5, 1H).

### Example 13. Also disclosed in the present application: (S)-3-Hydroxy-1-(1-hydroxy propyl-2-)-2-ethyl pyridine-4(1H)-one hydrochloride, Number: CN406.

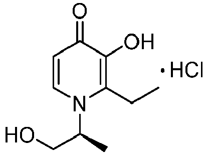

63.8 g 3-phenyloxy-2-ethyl-4H-pyran-4-one **(Example 2**) was dissolved in 150 mL n-butanol, then 41.6 g L-alaninol was added in. After thoroughly mixing, the solution was refluxed at 118°C for 36 h. After cooling and filtration, the residue was washed with ethyl acetate, resulting in white solid, which was then dissolved into 150 mL ethanol and 15 mL water, then it was hydrogenated and debenzylated with 5% Pd/C as catalyst, the solvent was removed under rotary evaporation, the residue was recrystalized with methanol and diethyl ether. The crude product was treated with HCl methanol solution to generate hydrochloride. After recrystallization, 53.21 g white solid was obtained with the yield of 83.4%. specific rotation [α]¹⁸_{D}= +23.73, ¹HNMR (DMSO-*d₆*): δ 1.16 (t, J=7.5, 3H), 1.44 (d, J=6.5, 3H), 3.05 (m, 2H), 3.73 (d, J=6, 2H), 4.84 (m, 1H), 7.47 (m, 1H), 8.41 (m, 1H).

### Example 14. (R)-3-hydroxy-1-(1-hydroxy-3-phenyl propyl-2-)-2-ethyl pyridine-4(1H)-one hydrochloride, Number: CN428.

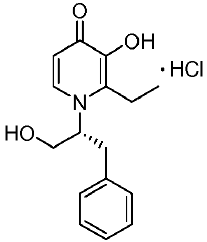

63.8 g 3-phenyloxy-2-ethyl-4H-pyran-4-one **(Example 1**) was dissolved in 150 mL n-butanol, then 83.7 g L-phenoanalinol was added in. After thoroughly mixing, the solution was refluxed at 118°C for 36 h. After cooling and filtration, products were separated by silica gel column chromatography with eluent ethanol: ethyl acetate= 1:40. After elution, light brown solid product was obtained after rotary evaporation, which was then dissolved into 150 mL ethanol and 15 mL water, then it was hydrogenated and debenzylated with 5% Pd/C as catalyst, the solvent was removed under rotary evaporation, the remaining solid was recrystallized with methanol and ether, leading to 21.85 g light yellow solid. The yield was 34.3%, specific rotation [α]¹⁸_{D}= +183.48, ¹HNMR (DMSO-*d₆*): δ 0.88 (t, J=7.5, 3H), 2.63 (dq, J₁=15, J₂=7.5, 1H), 2.80 (dq, J₁=15, J₂=7.5, 1H), 3.16 (dd, J₁=14, J₂=11, 1H), 3.25 (dd, J₁=14.5, J₂=5, 1H), 3.87 (m, 2H), 4.97 (m, 1H), 7.18 (m, 1H), 7.46 (m, 1H), 8.63 (d, J=7.5, 1H).

### Example 15. Also disclosed in the present application: (R)-3-Hydroxy-1-(1-hydroxy-3-propyl-2-)-2-methyl pyridine-4(1H)-one hydrochloride, Number: CN308.

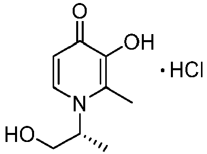

60 g 3-phenyloxy-2-methyl-4H-pyran-4-one **(Example 1**) was dissolved in 150 mL n-butanol, then 41.6 g D-alaninol was added in. After thoroughly mixing, the solution was refluxed at 118°C for 36 h. After cooling and filtration, the residue was washed with ethyl acetate to get white solid, which was then dissolved into 150 mL ethanol and 15 mL water, then it was hydrogenated and debenzylated with 5% Pd/C as catalyst. The solvent was removed under rotary evaporation, the residue was recrystallized with methanol and ether. The resulting crude product was then treated with HCl methanol solution to generate hydrochloride. After recrystallization, 47.25 g light yellow solid was obtained with the yield of 78.8%. specific rotation [α]¹⁸_{D}= -13.65, ¹HNMR (DMSO-*d₆*): δ 1.43 (d, J=7, 3H), 2.60 (s, 3H), 3.67 (dd, J₁=12, J₂=8, 1H), 3.74 (dd, J₁=12, J₂=4.5, 1H), 4.88 (m, 1H), 7.41 (m, 1H), 8.35 (d, J=7, 1H).

### Example 16. Also disclosed in the present application: (R)-3-Hydroxy-1-(1-hydroxy-3-propyl-2-)-2-ethyl pyridine-4(1H)-one hydrochloride, Number: CN408.

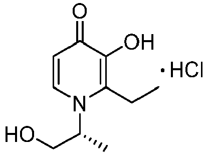

63.8 g 3-phenyloxy-2-methyl-4H-pyran-4-one **(Example 2**) was dissolved with 150 mL n-butanol, then 41.6 g D-alaninol was added in. After thoroughly mixing, the solution was refluxed at 118°C for 36 h. After cooling and filtration, the residue was washed with ethyl acetate to get white solid; which was then dissolved into 150 mL ethanol and 15 mL water, then it was hydrogenated and debenzylated with 5% Pd/C as catalyst, the solvent was removed under rotary evaporation, the resulting residue was recrystallized with methanol and diethyl ether. Then crude product was treated with HCl methanol solution to get hydrochloride. After recrystallization, 44.34 g light yellow solid was obtained with the yield of 69.5%. specific rotation [α]¹⁸_{D}= -27.45, ¹HNMR (DMSO-*d₆*): δ 1.16 (t, J=7.5, 3H), 1.43 (d, J=7, 3H), 3.01 (q, J=7.5, 2H), 3.72 (d, J=6, 2H), 4.78 (m, 1H), 7.14 (m, 1H), 8.27 (d, J=7, 1H).

### Example 17. Also disclosed in the present application: (R)-3-Hydroxy-1-(1-hydroxy-3-methyl butyl-2-)-2-methyl pyridine-4(1H)-one, Number: CN118.

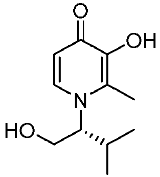

60 g 3-benzyloxy-2-methyl-4H-pyran-4-one **(Example 1)** was dissolved in 150 mL n-butanol, then 56.1 g L-valinol was added in. After thoroughly mixing, the solution was refluxed at 118°C for 36 h. After cooling and filtration, products were separated by silica gel column chromatography with eluent ethanol: ethyl acetate= 1:40. After elution, light yellow solid was obtained after rotary evaporation; which was then dissolved into 150 mL ethanol and 15 mL water, then it was hydrogenated and debenzylated with 5% Pd/C as catalyst, the solvent was removed under rotary evaporation, the remaining solid was recrystallized with methanol and ether, leading to 23.03 g white solid, the yield was 39.3%, the free alkali's ¹H NMR (DMSO-*d₆*) : δ 0.6 (d, J=7, 3H), 1.02 (d, J=6.5, 3H), 2.08 (m, 1H), 2.29 (s, 3H), 3.69 (dd, J₁=12, J₂=8, 1H), 3.78 (dd, J₁=11.5, J₂=3, 1H), 3.88 (m, 1H), 6.18 (d, J=7.5, 1H), 7.64 (d, J=7.5, 1H).

### Example 18. Also disclosed in the present application: (R)-3-Hydroxy-1-(1-hydroxy-3-methyl butyl-2-)-2-methyl pyridine-4(1H)-one hydrochloride, Number: CN138.

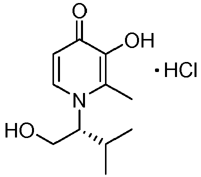

Treated (*R*)-3-Hydroxy-1-(1-hydroxy-3-methyl butyl-2-)-2-methyl pyridine-4(1*H*)-one **(Example 17)** with HCl methanol solution to form hydrochloride. The recrystallization later resulted in 19.66 g light yellow solid with the yield of 72.8%. specific rotation [α]¹⁸_{D}= +38.79, ¹HNMR (DMSO-*d₆*): δ 0.64 (d, J7, 3H), 1.08 (d, J=6.5, 3H), 2.23 (m, 1H), 2.58 (s, 3H), 3.77 (dd, J₁=12, J₂=8.5, 1H), 3.90 (dd, J₁=12, J₂=7, 1H), 4.42 (m, 1H), 7.40 (m, 1H), 8.35 (d, J=7, 1H).

### Example 19. Also disclosed in the present application: (R)-3-Hydroxy-1-(1-hydroxy-3-methyl butyl-2-)-2-ethyl pyridine-4(1H)-one, Number: CN218.

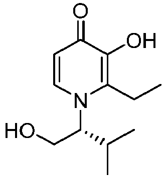

63.8 g 3-benzyloxy-2-ethanol-4H-pyran-4-one **(Example 2**) was dissolved in 150 mL n-butanol, then 56.1 g D-valinol was added in. After thoroughly mixing, the solution was refluxed at 118°C for 36 h. After cooling and filtration, products were separated by silica gel column chromatography with eluent ethanol: ethyl acetate= 1:40. After elution, light yellow solid was obtained after rotary evaporation; which was then dissolved into 150 mL ethanol and 15 mL water, then it was hydrogenated and debenzylated with 5% Pd/C as catalyst, the solvent was removed under rotary evaporation, the residue was recrystallized with methanol and ether, leading to 19.62 g light yellow solid, the yield was 31.4%, the free alkali's ¹HNMR (DMSO-*d₆*): δ 0.6 (d, J=6.5, 3H), 1.04 (d, J=7.5, 3H), 1.11 (t, J=7), 2.11 (m, 1H), 2.71 (dq, J₁=14.5, J₂=7, 1H), 2.81 (dq, J₁=14.5, J₂=7, 1H), 3.75 (m, 2H), 3.84 (m, 1H), 6.17 (d, J=7.5, 1H), 7.63 (d, J=7.5, 1H).

### Example 20. Also disclosed in the present application: (R)-3-Hydroxy-1-(1-hydroxy-3-methyl butyl-2-)-2-ethyl pyridine-4(1H)-one hydrochloride, Number: CN418.

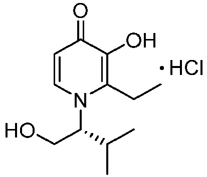

Treated (*R*)-3-Hydroxy-1-(1-hydroxy-3-methyl butyl-2-)-2-ethyl pyridine-4(1*H*)-one **(Example 19)** with HCl methanol solution to form hydrochloride. After recrystallization later, 17.26 g light yellow solid was obtained with the yield of 75.7%. specific rotation [α]¹⁸_{D}= +50.71, ¹HNMR (DMSO-*d₆*): δ 0.64 (d, J=6.5, 3H), 1.10 (d, J=7.5, 3H), 1.17 (t, J=7.5, 3H), 2.25 (m, 1H), 3.04 (m, 2H), 3.82 (dd, J₁=12, J₂=7.5, 1H), 3.88 (dd, J₁=12, J₂=3.5, 1H), 4.39 (m, 1H), 7.45 (m, 1H), 8.36 (d, J=7.5, 1H).

### Example 21. (S)-3-Hydroxy-1-(1-hydroxy-3-phenyl propyl-2-)-2-methyl pyridine-4(1H)-one hydrochloride, Number: CN326.

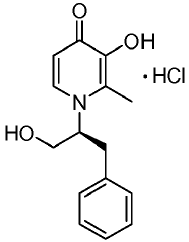

60 g 3-phenyloxy-2-methyl-4H-pyran-4-one **(Example 1**) was dissolved in 150 mL n-butanol, then 83.7 g L-phenoalaninol was added in. After thoroughly mixing, the solution was refluxed at 118°C for 36 h. After cooling and filtration, products were separated by silica gel column chromatography with eluent ethanol: ethyl acetate= 1:40. After elution, light brown solid product was obtained after rotary evaporation; which was then dissolved into 150 mL ethanol and 15 mL water, then it was hydrogenated and debenzylated with 5% Pd/C as catalyst,the solvent was removed under rotary evaporation, the resulting residue was recrystallized with methanol and ether, leading to 23.25 g white solid, the yield was 38.8%, specific rotation [α]¹⁸_{D}=-162.76, ¹HNMR (DMSO-*d₆*): δ 2.30 (s, 3H), 3.13 (dd, J₁=14, J₂=10.5, 1H), 3.28 (dd, J₁=14, J₂=5, 1H), 3.87 (m, 2H), 5.08 (m, 1H), 7.20 (m, 5H), 7.47 (dd, J₁=9, J₂=7, 1H), 8.61 (t, J=7.5, 1H).

### Example 22. (S)-3-Hydroxy-1-(1-hydroxy-3-phenyl propyl-2-)-2-ethyl pyridine-4(1H)-one hydrochloride, Number: CN426.

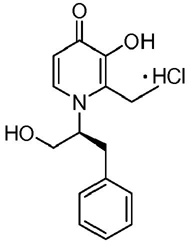

63.8 g 3-phenyloxy-2-methyl-4H-pyran-4-one **(Example 2)** was dissolved in 150 mL n-butanol, then 83.7 g L-phenylalaninol was added in. After thoroughly mixing, the solution was refluxed at 118°C for 36 h. After cooling and filtration, products were separated by silica gel column chromatography with eluent ethanol: ethyl acetate= 1:40, After elution, light brown solid product was obtained after rotary evaporation; which was then dissolved into 150 mL ethanol and 15 mL water, then it was hydrogenated and debenzylated with 5% Pd/C as catalyst, the solvent was removed under rotary evaporation, the resulting residue was recrystallized with methanol and diethyl ether, leading to 16.50 g white solid, the yield was 25.9%. specific rotation [α]¹⁸_{D}= -189.29, ¹HNMR (DMSO-*d₆*): δ 0.88 (t, J=7.5, 3H), 2.63 (dq, J₁=15, J₂=7.5, 1H), 2.80 (dq J₁=15, J₂=7.5, 1H), 3.16 (dd, J₁=14, J₂=5, 1H), 3.26 (dd, J₁=14, J₂=5, 1H), 3.88 (m, 2H), 4.97 (m, 1H), 7.18 (m, 5H), 7.5 (m, 1H), 8.65 (m, 1H).

### Example 23. Also disclosed in the present application: 3-Hydroxy-1-((2S)-1-hydroxy-3-methyl pentyl-2-)-2-ethyl pyridine-4(1H)-one hydrochloride, Number: CN436.

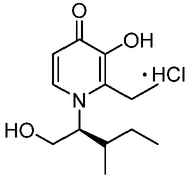

63.8 g 3-phenyloxy-2-ethyl-4H-pyran-4-one **(Example 2**) was dissolved in 150 mL n-butanol, then 64.9 g L-isoleucinol was added in. After thoroughly mixing, the solution was refluxed at 118°C for 36 h. After cooling and filtration, products were separated by silica gel column chromatography with eluent ethanol: ethyl acetate= 1:40, after elution, light yellow solid product was obtained after rotary evaporation; which was then dissolved into 150 mL ethanol and 15 mL water, then it was hydrogenated and debenzylated with 5% Pd/C as catalyst, the solvent was removed under rotary evaporation, the resulting residue was recrystallized with methanol and ether, leading to 18.04 g white solid. The yield was 28.3%. specific rotation [α]¹⁸_{D}= -38.29, ¹HNMR (DMSO-*d₆*): δ 0.77 (t, J=7.5, 3H), 0.90 (m, 2H), 1.05 (d, J=6.5, 3H), 1.16 (t, J=7.5, 3H), 2.00 (m, 1H), 2.96 (dq, J₁=15.5, J₂=7.5, 1H), 3.08 (dq, J₁=15.5, J₂=7.5, 1H), 3.83 (dd, J₁=12.5, J₂=8, 1H), 3.88 (dd, J₁=12, J₂=4.5, 1H), 4.45 (m, 1H), 7.42 (m, 1H), 8.35 (d, J=7.5, 1H).

### Example 24. Also disclosed in the present application: (S)-3-Hydroxy-1-(1-hydroxy-4-methyl pentyl-2-)-2-methyl pyridine-4(1H)-one hydrochloride, Number: CN346.

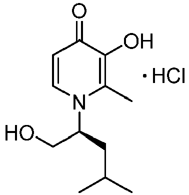

2.46 g 3-phenyloxy-2-methyl-4H-pyran-4-one **(Example 1**) was dissolved with 8.5 mL n-butanol, then 2.0 g L-leucinol was added in. After thoroughly mixing, the solution was refluxed at 118°C for 36 h, after cooling and filtration, products were separated by silica gel column chromatography with eluent ethanol: ethyl acetate= 1:30. After elution, light brown oily liquid was obtained after rotary evaporation, the solid was then dissolved into 50 mL ethanol and 5 mL water, then it was hydrogenated and debenzylated with 5% Pd/C as catalyst, the solvent was removed under rotary evaporation, the resulting residue was recrystallized with methanol and diethyl ether, leading to 1.24 g white solid. The yield was 50.4%. specific rotation [α]¹⁸_{D}= -15.56.

### Example 25. Also disclosed in the present application: (S)-3-Hydroxy-1-(1-hydroxy-4-methyl pentyl-2-)-2-ethyl pyridine-4(1H)-one hydrochloride, Number: CN446.

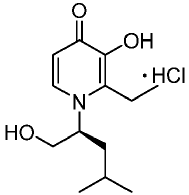

2.61 g 3-phenyloxy-2-ethyl-4H-pyran-4-one **(Example 2**) was dissolved in 8.5 mL n-butanol, then 2.0 g L-leucinol was added in, after thoroughly mixing, the solution was refluxed at 118°C for 36 h, after cooling and filtration, products were separated by silica gel column chromatography with eluent ethanol: ethyl acetater= 1:30. After elution, light brown oily liquid was obtained after rotary evaporation, which was then dissolved into 50 mL ethanol and 5 mL water, then it was hydrogenated and debenzylated with 5% Pd/C as catalyst, the solvent was removed under rotary evaporation, the resulting residue was recrystallized with methanol and ether, leading to 1.25 g light yellow solid, the yield was 48.0%, specific rotation [α]¹⁸_{D}= -14.36.

### Example 26. Also disclosed in the present application: 3-Hydroxy-1-(1,3-dihydroxy propyl-2-)-2-methyl pyridine-4(1H)-one hydrochloride, Number: CN350.

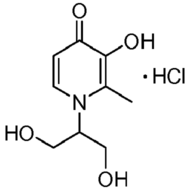

12.65 g 3-phenyloxy-2-methyl-4H-pyran-4-one (Example 1) was dissolved in 8.5 mL n-butanol, then 8.0 g D-leucinol was added in, after thoroughly mixing, the solution was refluxed at 118°C for 36 h, after cooling and filtration, products were separated by silica gel column chromatography with eluent ethanol: ethyl acetate= 1:20. After elution, light brown oily liquid was obtained after rotary evaporation, the solid was then dissolved into 50 mL ethanol and 5 mL water, then it was hydrogenated and debenzylated with 5% Pd/C as catalyst, the solvent was removed under rotary evaporation, the resulting residue was recrystallized with methanol and ether, leading to 7.43 g white solid, the yield was 58.8%.

### Example 27. Also disclosed in the present application: 3-Hydroxy-1-(1,3-dihydroxy propyl-2-)-2-ethyl pyridine-4(1H)-one hydrochloride, Number: CN450.

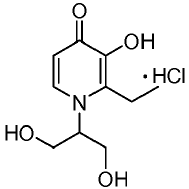

11.95 g 3-phenyloxy-2-ethyl-4H-pyran-4-one (Example **2**) was dissolved in 8.5 mL n-butanol, then 7.09 g D-leucinol was added in, after thoroughly mixing, the solution was refluxed at 118°C for 36 h, after cooling and filtration, products were separated by silica gel column chromatography with eluent ethanol: ethyl acetate= 1:20. After elution, light brown oily liquid was obtained after rotary evaporation, the solid was then dissolved into 50 mL ethanol and 5 mL water, then it was hydrogenated and debenzylated with 5% Pd/C as catalyst, the solvent was removed under rotary evaporation, the resulting residue was recrystallized with methanol and ether, leading to 2.77 g white solid, yield is 43.2%.

### Example Part (Two). Biological effect of chiral 3-hydroxy pyridine-4-one derivatives.

### Example 1 Preparation of ⁵⁹Fe-Ferritin

According to methods described by Pippard's research group [Blood 58: 685-692], use ⁵⁹Fe to label ferritin in rat liver. Isolate ⁵⁹Fe-labelled ferritin by the method described by Bjorklid and Helgeland. In the preparation process, the rats were injected intraperitoneally with iron dextran, and intravenous administration of red blood cells, in order to keep the plasma transferrin saturated. This method ensures that most of ⁵⁹Fe (III) is absorbed by the liver, so ⁵⁹Fe-ferritin with high specific radioactivity can be obtained.

### Example 2 Preparation of animals.

Performance of one shot intraperitoneal injection of 15mg iron dextran to 350∼400 g rats. After one week, jugular vein cannula is performed and then a 24 h's rest. Then the second day, each rat is given packed red cells (2 mL/100g). After 24 h, each rat is injected via the jugular vein cannula with 200 µCi ⁵⁹FeCl solution (Amersham Life Science). ⁵⁹FeCl₃ (1mCi) is diluted 0.5 mL citrate (50mM, pH 2.5). Each experiment employs 5 rats, total radiation is 1mCi. 24 h after isotopically labeled injection, the livers were removed from rats.

### Example 3 Purification of ⁵⁹Fe-Ferritin.

Homogenization of liver is processed in 4 vol. (w/v) 0.25M acetate buffer. The homogenate was heated to 75°C with stirring for 10min. After cooling to 4°C, remove solid protein by centrifugation at 2500 g for 15 min. Treat the supernatant that contains tissue iron protein with half saturated (NH₄)₂SO₄, while cooling in ice water for 30 min. The precipitate obtained by centrifugation at 3500 g at 4°C for 15 min, is dissolved in 0.25M pH=4.8 acetate buffer. Undissolved precipitate is removed through a Pd-10 column (Sephadex®, G-25M, Pharmacia Biotech). The resulting solution is centrifuged at 100,000 g at 4°C for 2 h. The final precipitate is dissolved in 0.05M pH=7.4 phosphate buffer, and stored at 4°C. The purity of ⁵⁹Fe-ferritin and normal Ferritin (Sigma Chemical Co.) are compared by electrophoresis. Iron concentration of ferritin is tested by colorimetry [Biochem Mol. Biol Int 35(3): 635-631].

### Example 4. Determination of iron concentration via colorimetry.

Separate 100 µL 10M HCl into two parts, stored in plastic containers respectively, of which one has 50 µL ⁵⁹Fe-Ferritin solution(ctn 1), the other is blank (ctn 2). Two samples are incubated at 37°C overnight, to hydrolyze the iron proteins and release the iron ions. Then dilute each sample with 10 mL deionized water, wherein, 1.7 mL solution is transferred into two other containers (3, bottle 4). Add 300 µL 800mM nitrilotriacetic acid into each containers (bottle 3, bottle 4), let stand at room temperature for 30 min. Then the sample was subjected to ultrafiltration through the porous membrane (Cetricon-30), and then centrifuge at 25°C at 4000g for 60min. Add 1mL filtrate to a container which has 250 µL 120mM Thioglycollic acid and 250 µL 60mM bathophenanthroline disulfite, then add disodium salt. 30 min later, absorbance is tested under 537nm. For concentration of iron ion in the range of 2.5∼50 µM (0.1M HCl as solvent), draw a six-point standard curve through the method described above, it can be used as quantitative reference of iron concentration in ferritin.

### Example 5. Test of biliary excretion of iron in normal rat.

250∼300 g Wistar rat with previous starve treatment are kept in Nalgene plastic bio-metabolism cage, with sufficient water supply for SD rat. They are anesthetized by pentobarbital sodium (intraperitoneal injection, 50mg/kg), Use PE50 size 22 catheter to insert into bile about 2cm deep, and ligate strong. Then pierce a transculaneous needle through the shoulder area near abdominal incision of rat, and transfer the bile duct cannula out of the skin of rats by this needle. The lead portion of the catheter from the shoulder connects to the rotary shaft of a metal torque transfer pump, which is fixed to the rats' suit. The catheter finally access Gilson trace collector by the liquid shaft on metabolic cages. This system allows the rat to move freely in the cage while continuously collect bile samples. 24 h urea is also collected with disposable plastic tubes. Various Iron (III) chelators are intragastrically administered to the rat, blank group is given same volume water through intragastric administration. Bile samples are collected once every 12 h with one-time disposable plastic tube (Portex Ltd.). A light anesthesia by 2.5 mg/kg fentanyl and 0.08 mg/kg fluanisone (Hypnorm®) is performed while collecting. Concentration of iron is tested by the chromatometry method described above.

### Example 6. Test method of Biological effect of iron chelators over ⁵⁹Fe-Ferritin/ Rat.

Starved rat was injected ⁵⁹Fe-Ferritin via tail vein, in order to label rat liver cells with ⁵⁹Fe. After 1 h, each rat is given an iron chelator via intragastric injection. Blank group is administered with water by lavage. Each rat is placed in a metabolic cage, collect urine and feces. All rats are given free access to food after oral administration 1h later, and are free to drink water during the whole experiment. Test ends after 24 h of the tail vein injection of ⁵⁹Fe-Ferritin. All rats are killed by the cervical dislocation, then the liver and gastrointestinal system (including its content and feces) are collected for ⁵⁹Fe quantification. Calculation for iron excretion ability of iron chelators with following formula:
(1) Iron Excretion(%)=⁵⁹Fe Activity_{(intestinal track & feces)}/(⁵⁹Fe Activity_{(intestinal tracks & feces)}+ ⁵⁹Fe Activity₍ₗᵢᵥₑᵣ₎)×100%

**Table 1 Iron excretion ability of taking chiral 3-hydroxypyridine-4-one orally on rat model (n=5).**

| Chemical number | Formula | Iron Excretion (%) |
|---|---|---|
| CP20 | 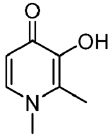 | 11.4 |
| CP102 | 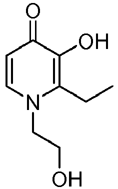 | 17.0 |
| CN306 | 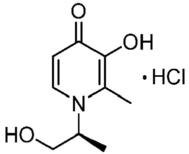 | 11.9 |
| CN308 | 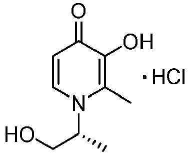 | 6.5 |
| CN206 | 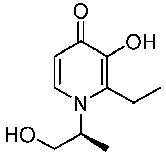 | 16 |
| CN408 | 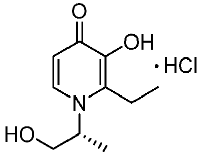 | 10.6 |
| CN116 | 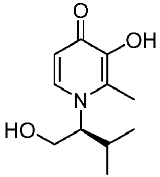 | 18.7 |
| CN318 | 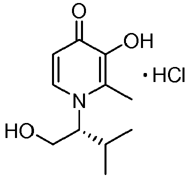 | 8.0 |
| CN416 | 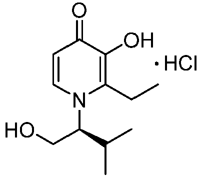 | 7.9 |
| CN418 | 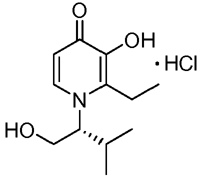 | 10.6 |
| CN326 | 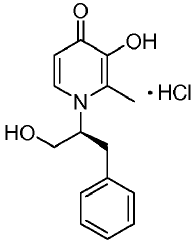 | 12.8 |
| CN128 | 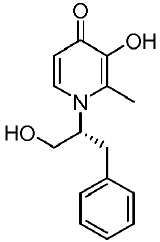 | 24.8 |
| CN426 | 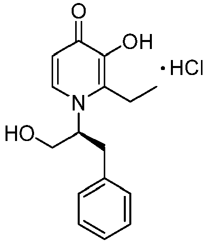 | 13.1 |
| CN228 | 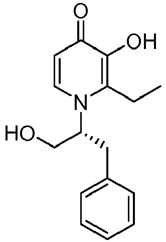 | 22.3 |
| CN336 | 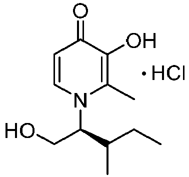 | 10.8 |
| CN436 | 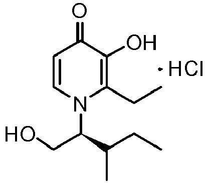 | 5.8 |

| | | |
|---|---|---|
| Note: n represents the number of tested animals for each compound. | | |

In vivo rat test shows, the series of chiral 3-hydroxypyridin-4-one derivatives described in this patent has good iron excretion ability, some preferred compounds have superior iron excretion ability than the market available iron chelator CP20, i.e. the iron excretion ability of CN116, CN206, CN128 and CN228 is several times better than CP20 administered in equimolar amounts.

### Example Part (Three). Acute toxicity test of some chiral 3-hydroxypyridin-4-ones.

### Example 1. Method

Prepare suspension of maximum solubility of each compound with 0.5% CMC-Na. Maximum oral dosage to mice is 0.4 mL/10g. Blank group took the same volume 0.5% CMC-Na solution orally (blank group and test group has 4 mice each, 2 female and 2 male). If test group shows fatality reaction, reduce the dose of drug administered, until there is no fatality. Monitor reaction of animal right after drug administration, including appearance, activities, mental states, appetites, urea/feces and its color, fur, nose, eye, mouth, if there are any abnormal secretions and death situation, give timely autopsy to dead animals. If any obvious pathology of organs is found, perform histopathological inspection. Every inspection should be performed every 30∼60 min in the 1^{st} day, then check once a day, record animal toxicity and death distribution.

**Table 2. Maximum dosage of a few 3-hydroxypyridin-4-one derivatives to mice.**

| Compound Number | Conc. (g/mL) | Volume(mL) | Dosage(g/kg) | Mole dosage(mmol/kg) |
|---|---|---|---|---|
| CP20 | 0.06 | 0.5 | 1.50 | 10.7 |
| CP102 | 0.20 | 0.4 | 4.00 | 21.8 |
| CP106 | 0.20 | 0.5 | 5.00 | 27.3 |
| CN108 | 0.10 | 0.5 | 2.50 | 13.6 |
| CN206 | 0.15 | 0.5 | 3.75 | 19.0 |
| CN208 | 0.15 | 0.5 | 6.25 | 21.7 |

Acute toxicity test in mice shows, in vivo toxicity of the series of chiral 3-hydroxypyridin-4-one derivatives described in this patent is low, maximum lethal dose of some compounds was significantly larger than control drug CP20. Therefore, a smaller cure dosage compared to CP20, its toxicity will be much lower than CP20.

## Claims

1. A chiral 3-hydroxypyridin-4-one derivative or salts thereof having the following general formula I : 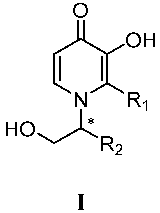 **characterised in that** the carbon atom marked with "*" is chiral,
R₂ represents aralkyl, or heterocyclic aralkyl; wherein
- the aralkyl refers to a strand of alkyl that contains at least one hydrogen atom, wherein a hydrogen atom attached to the terminal carbon is substituted by an aryl, comprising benzyl, p-chlorobenzyl, o-fluorobenzyl, phenethyl, p-methylbenzyl, p-dimethylamino benzyl, p-diethylamino benzyl, p-cyanobenzyl or p-pyrrolidinyl benzyl;
- The heterocyclic aralkyl refers to a strand of alkyl that contains at least one hydrogen atom, wherein a hydrogen atom attached to the terminal carbon atom is substituted by an aromatic heterocycle, wherein the aromatic heterocycle has one or more substituents selected from alkyl, halogen, trifluoromethyl, carboxyl, alkoxyacyl, N-alkylamino, N,N-dialkylamino or pyrrolidinyl; and
- R1 represents alkyl, hydroxy alkyl, mercapto alkyl, haloalkyl, alkoxy, alkylthiol, haloalkoxy, haloalkylthiol, carboxyl alkyl, alkoxyacyl-alkyl, R3R4N-C(O)-alkyl, heterocycloalkyl, unsubstituted aryl, aroyl, aralkyl, unsubstituted heterocyclic aryl or heterocyclic aralkyl, wherein
(i) the alkyl represents methyl, ethyl, n-propyl, isopropyl, n-butyl, isobutyl, n-pentyl, n-hexyl or n-heptyl;
(ii) the hydroxy alkyl is an alkyl substituted by hydroxy, wherein hydroxyl is at the terminal of alkyl or in the middle of alkyl, and comprises 3-hydroxypropyl, 2-hydroxypropyl, 1-hydroxypropyl or 1-hydroxy-2-methylpropyl;
(iii)the mercapto alkyl refers to a group in which oxygen atom in hydroxy alkyl is substituted by a sulfur atom;
(iv)the halogen alkyl refers to alkyl substituted by a halogen atom selected from chlorine or fluorine, or substituted by trichloro or trifluoro, and halogen substitution site is at the terminal or in the middle of alkyl;
(v) the alkoxy group refers to a group formed by alkyl and oxygen atom which is covalently linked to the substitution site via an ether linkage, and comprises methoxy, oxethyl, n-propoxy, isopropoxy, n-butoxy, isobutoxy, sec-butoxy, tert-butoxy, n-pentyloxy, isopentyloxy;
(vi) the alkylthiol group is a group in which the oxygen atom in alkoxy is substituted by sulfur atom;
(vii) the haloalkoxy refers to an alkoxy substituted by a halogen atom selected from chlorine, or fluorine, or substituted by trichloro or trifloro;
(viii) the haloalkylthiol refers to a group in which oxygen atom in haloalkoxy is substituted by sulfur atom;
(ix) the carboxyl alkyl refers to an alkyl substituted by carboxyl including a single carboxyl substitution, or multi-carboxyl substitution, and the substitution site is at the terminal or in the middle of the alkyl;
(x) the alkoxyacyl refers to "alkyl-O-C(O)-", and comprises methoxycarbonyl, ethoxycarbonyl, n-propoxycarbonyl, isopropoxycarbonyl, isobutoxycarbonyl, isobutoxycarbonyl, sec-butoxycarbonyl, tert-butoxycarbonyl, n-pentyloxycarbonyl, or isopentyloxycarbonyl;
(xi) R₃ and R₄ in R₃R₄N-C(O)-alkyl are same or different, and represent hydrogen, alkyl, hydroxy alkyl, alkoxy-alkyl, hydroxy alkoxy-alkyl, amino-alkyl, N-alkylamino-alkyl, N,N-dialkylamino-alkyl, N-hydroxyalkylamino-alkyl, N,N-dihydroxyalkylamino-alkyl, or a nitrogen-containing alicyclic ring formed by connecting two terminals of alkyl with a nitrogen atom;
(xii) the heterocyclic alkyl refers to a ring structure containing 3-8 atoms, wherein there is at least one heteroatom selected from oxygen, nitrogen or sulfur atom;
(xiii) the-unsubstituted aryl refers to an unsubstituted phenyl;
(xiv) the aroyl refers to "aryl-C(O)-", and comprises benzoyl, toluoyl, naphthoyl or p-methoxy benzoyl;
(xv) the aralkyl refers to a strand of alkyl that contains at least one hydrogen atom wherein a hydrogen atom attached to the terminal carbon is substituted by an aryl, and comprises benzyl, *p*-chlorobenzyl, *o*-fluorobenzyl, phenethyl, *p*-methylbenzyl, *p*-dimethylamino benzyl, *p*-diethylamino benzyl, *p*-cyanobenzyl or *p*-pyrrolidinyl benzyl;
(xvi) the heterocyclic aryl refers to an aromatic ring containing a heteroatom, and comprises aromatic heterocycle-alkyl; the aromatic heterocycle refers to an aromatic ring containing 3-7 atoms wherein there is at least one heteroatom, and comprises pyrrolidinyl, imidazolyl, triazolyl, tetrazolyl, oxygen-nitrogen cyclopentadienyl, thiazolyl, furanyl, thiophenyl, pyridyl, pyrazinyl, thiazinyl, pyranyl, or pyrimidyl; the aromatic heterocycle is unsubstituted;
(xvii)the heterocyclic aralkyl refers to a strand of alkyl that contains at least one hydrogen atom wherein a hydrogen atom attached to the terminal carbon atom is substituted by an aromatic heterocycle; and the aromatic heterocycle has one or more substituents selected from alkyl, halogen, trifluoromethyl, carboxyl, alkoxyacyl, N-alkylamino, N,N-dialkylamino or pyrrolidinyl.

2. The chiral 3-hydroxypyridin-4-one derivative or salts thereof according to claim 1 ,wherein the aryl is an unsubstituted phenyl or naphthyl, or a phenyl substituted by alkyl, alkoxy, hydroxy, halogen, carboxyl, alkloxyacyl, N,N-dialkylamino or heterocyclic alkylacyl; wherein N-alkylamino is n-propylamino, n-butylamino, isopropylamino, isobutylamino, or N-hydroxy-ethylamino; and N,N-dialkylamino has same or different alkyl substituents and comprises N,N-dimethylamino, N,N-diethylamino, N,N-methylethylamino, N-methyl-N-morpholino-ethylamino, N-methyl-N-hydroxyethylamino or N-methyl-N-anilino.

3. The chiral 3-hydroxypyridin-4-one derivative or salts thereof according to claims 1 or 2, wherein the salts are formed by a compound of formula **I** and an acid selected from strong inorganic acid, an alkyl carboxylic acid, saturated or unsaturated dicarboxylic acid or methyl or p-toluenesulfonic acid.

4. The chiral 3-hydroxypyridin-4-one derivative or salts thereof according to claim 3, wherein the strong inorganic acid is a mineral acid, and comprises sulfonic acid, phosphoric acid or hydrochloric acid; the alkyl carboxylic acid comprises acetic acid; the saturated or unsaturated dicarboxylic acid comprises tartaric acid or citric acid.

5. The chiral 3-hydroxypyridin-4-one derivative or salts thereof according to any of claims 1 to 4, wherein
(a) R₁ in formula I is methyl, and compound is shown in formula **II**: 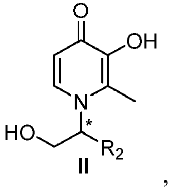 or
(b) R₁ in formula I is ethyl, and compound is shown in formula **III:** 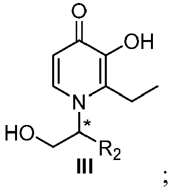
wherein the carbon atom marked with "*" is chiral and R₂ is defined as that in formula **I**.

6. The chiral 3-hydroxypyridin-4-one derivative or salts thereof according to claim 5 selected from Compound III-c: (*R*)-3-Hydroxy-1-(1-hydroxy-3-phenyl propyl-2-)-2-ethyl pyridine-4(1*H*)-one or Compound II-c: (*R*)-3-Hydroxy-1-(1-hydroxy-3-phenyl propyl-2-)-2-methyl pyridine-4(1*H*)-one.

7. A method for preparing the chiral 3-hydroxypyridin-4-one derivative or salts thereof according to any of claims 1 to 6, wherein said method comprises the following steps: reacting compound **V** with compound **VI,** thereby forming compound **VII** which is benzyl protected at 3 position, then reacting compound **VII** with compound **VIII,** thereby forming compound **IX**, and finally, in the presence of Pd/C catalyst, deprotecting compound **IX** via a hydrogenation reduction, thereby forming compound of formula **I,** and the reaction scheme is as follows:

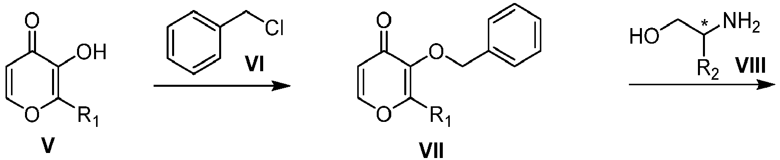

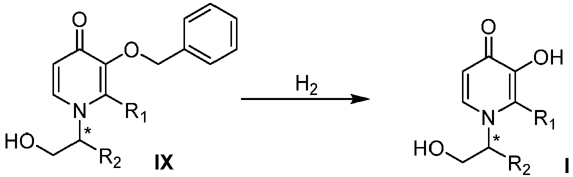

8. The method according to claim 7 further comprising the step of and reacting compound of formula I with an inorganic or organic acid HX to form corresponding salts, and the reaction scheme is as follows:

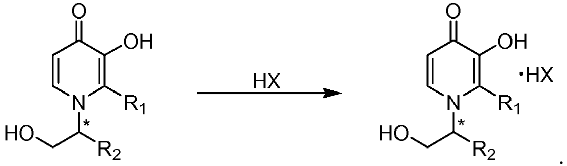

9. The chiral 3-hydroxypyridin-4-one derivative or salts thereof according to any of claims 1 to 6 for use as a medicament for the treatment of a disease selected from the group consisting of thalassemia, iron mediated tissue lesions in neurodegenerative diseases and onco-diseases.

10. The chiral 3-hydroxypyridin-4-one derivative or salts thereof for use according to Claim 9, wherein the chiral 3-hydroxypyridin-4-one derivative or salts thereof according to any of claims 1 to 6 is formulated into a formulation comprising a liquid preparation or a solid preparation, and the mode of administration is oral administration or administration by injection.

11. A medicament comprising the chiral 3-hydroxypyridin-4-one derivative or salts thereof according to any of claims 1 to 6.

12. The medicament according to claim 11, wherein the medicament comprises the chiral 3-hydroxypyridin-4-one derivative or salts thereof as active ingredient and a pharmaceutically acceptable carrier or excipient.

13. The medicament according to claims 11 or 12, wherein the medicament is formulated in liquid form or solid form.

14. The medicament according to claims 11 to 13, for use in the treatment of a disease selected from the group consisting of thalassemia, iron mediated tissue lesions in neurodegenerative diseases and onco-diseases.

## Patentansprüche

1. Chirales 3-Hydroxypyridin-4-on-Derivat oder Salze davon mit der folgenden allgemeinen Formel **I:** 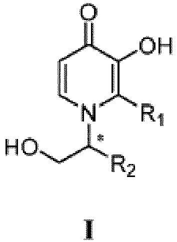 **dadurch gekennzeichnet, dass** das mit "*" markierte Kohlenstoffatom chiral ist,
- R₂ Aralkyl oder heterocyclisches Aralkyl darstellt; wobei:
- sich das Aralkyl auf einen Alkylstrang bezieht, der zumindest ein Wasserstoffatom enthält, wobei ein an den terminalen Kohlenstoff angebundenes Wasserstoffatom durch ein Aryl substituiert ist, umfassend Benzyl, p-Chlorbenzyl, o-Fluorbenzyl, Phenethyl, p-Methylbenzyl, p-Dimethylaminobenzyl, p-Diethylaminobenzyl, p-Cyanobenzal oder p-Pyrrolidinylbenzyl;
- sich das heterocyclische Aralkyl auf einen Alkylstrang bezieht, der zumindest ein Wasserstoffatom enthält, wobei ein an das terminale Kohlenstoffatom angebundenes Wasserstoffatom durch einen aromatischen Heterocyclus substituiert ist, wobei der aromatische Heterocyclus einen oder mehrere Substituenten aufweist, die aus Alkyl, Halogen, Trifluormethyl, Carboxyl, Alkoxyacyl, N-Alkylamino, N,N-Dialkylamino oder Pyrrolidinyl ausgewählt sind; und
- R1 Alkyl, Hydroxyalkyl, Mercaptoalkyl, Haloalkyl, Alkoxy, Alkylthiol, Haloalkoxy, Haloalkylthiol, Carboxylalkyl, Alkoxyacyl-alkyl, R3R4-C(O)-Alkyl, Heterocycloalkyl, unsubstituiertes Aryl, Aroyl, Aralkyl, unsubstituiertes heterocyclisches Aryl oder heterocyclisches Aralkyl darstellt, wobei
(i) das Alkyl Methyl, Ethyl, n-Propyl, Isopropyl, n-Butyl, Isobutyl, n-Pentyl, n-Hexyl oder n-Heptyl darstellt;
(ii) das Hydroxyalkyl ein durch Hydroxy substituiertes Alkyl ist, wobei Hydroxyl am Terminus von Alkyl oder in der Mitte von Alkyl vorhanden ist und 3-Hydroxypropyl, 2-Hydroxypropyl, 1-Hydroxypropyl oder 1-Hydroxy-2-methylpropyl umfasst;
(iii) sich das Mercaptoalkyl auf eine Gruppe bezieht, in der ein Sauerstoffatom in Hydroxyalkyl durch ein Schwefelatom substituiert ist;
(iv) sich das Halogenalkyl auf ein Alkyl bezieht, das durch ein Halogenatom substituiert ist, das aus Chlor oder Fluor ausgewählt ist, oder das durch Trichlor oder Trifluor substituiert ist, und wobei eine Halogensubstitutionsstelle am Terminus oder in der Mitte von Alkyl vorhanden ist;
(v) sich die Alkoxygruppe auf eine Gruppe bezieht, die durch Alkyl und ein Sauerstoffatom gebildet ist, das über eine Etherverknüpfung kovalent mit der Substitutionsstelle verknüpft ist, und die Methoxy, Oxethyl, n-Propoxy, Isopropoxy, n-Butoxy, Isobutoxy, sec-Butoxy, tert-Butoxy, n-Pentyloxy und Isopentyloxy umfasst;
(vi) die Alkylthiolgruppe eine Gruppe ist, in der das Wasserstoffatom in Alkoxy durch ein Schwefelatom substituiert ist;
(vii) sich das Haloalkoxy auf ein Alkoxy bezieht, das durch ein Halogenatom substituiert ist, das aus Chlor oder Fluor ausgewählt ist oder das durch Trichlor oder Trifluor substituiert ist;
(viii) sich das Haloalkylthiol auf eine Gruppe bezieht, in der ein Sauerstoffatom in Haloalkoxy durch ein Schwefelatom substituiert ist;
(ix) sich das Carboxylalkyl auf ein Alkyl bezieht, das durch Carboxyl substituiert ist, das eine einzelne Carboxylsubstitution oder eine Mehrfachcarboxylsubstitution enthält, und wobei die Substitutionsstelle am Terminus oder in der Mitte des Alkyls ist;
(x) sich das Alkoxyacyl auf "Alkyl-O-C(O)-" bezieht und Methoxycarbonyl, Ethoxycarbonyl, n-Propoxycarbonyl, Isopropoxycarbonyl, Isobutoxycarbonyl, Isobutoxycarbonyl, sec-Butoxycarbonyl, tert-Butoxycarbonyl, n-Pentyloxycarbonyl oder Isopentyloxycarbonyl umfasst;
(xi) R₃ und R₄ in R₃R₄N-C(O)-Alkyl gleich oder unterschiedlich sind und Wasserstoff, Alkyl, Hydroxyalkyl, Alkoxy-alkyl, Hydroxyalkoxy-alkyl, Amino-alkyl, N-Alkylaminoalkyl, N,N-Dialkylamino-alkyl, N-Hydroxyalkylamino-alkyl, N,N-Dihydroxyalkylamino-alkyl oder einen stickstoffhaltigen alicyclischen Ring darstellen, der durch Verbinden von zwei Termini von Alkyl mit einem Stickstoffatom gebildet ist;
(xii) sich das heterocyclische Alkyl auf eine Ringstruktur bezieht, die 3 bis 8 Atome enthält, wobei zumindest ein Heteroatom vorhanden ist, das aus Sauerstoff-, Stickstoff- oder Schwefelatom ausgewählt ist;
(xiii) sich das unsubstituierte Aryl auf ein unsubstituiertes Phenyl bezieht;
(xiv) sich das Aroyl auf "Aryl-C(O)-" bezieht und Benzoyl, Toluoyl, Naphthoyl oder p-Methoxybenzoyl umfasst;
(xv) sich das Aralkyl auf einen Alkylstrang bezieht, der zumindest ein Wasserstoffatom enthält, wobei ein an den terminalen Kohlenstoff angebundenes Wasserstoffatom durch ein Aryl substituiert ist, umfassend Benzyl, *p*-Chlorbenzyl, *o*-Fluorbenzyl, Phenethyl, *p-*Methylbenzyl, *p*-Dimethylaminobenzyl, *p*-Diethylaminobenzyl, *p*-Cyanobenzyl oder *p-*Pyrrolidinylbenzyl;
(xvi) sich das heterocyclische Aryl auf einen aromatischen Ring bezieht, der ein Heteroatom enthält und der aromatisches Heterocyclus-alkyl umfasst; wobei sich der aromatische Heterocyclus auf einen aromatischen Ring bezieht, der 3 bis 7 Atome enthält, wobei zumindest ein Heteroatom vorhanden ist, und Pyrrolidinyl, Imidazolyl, Triazolyl, Tetrazolyl, Sauerstoff-Stickstoff-Cyclopentadienyl, Thiazolyl, Furanyl, Thiophenyl, Pyridyl, Pyrazinyl, Thiazinyl, Pyranyl oder Pyrimidyl; wobei der aromatische Heterocyclus unsubstituiert ist;
(xvii) sich das heterocyclische Aralkyl auf einen Alkylstrang bezieht, der zumindest ein Wasserstoffatom enthält, wobei ein an das terminale Kohlenstoffatom angebundene Wasserstoffatom durch einen aromatischen Heterocyclus substituiert ist, und wobei der aromatische Heterocyclus einen oder mehrere Substituenten aufweist, die aus Alkyl, Halogen, Trifluormethyl, Carboxyl, Alkoxyacyl, N-Alkylamino, N,N-Dialkylamino oder Pyrrolidinyl ausgewählt sind.

2. Chirales 3-Hydroxypyridin-4-on-Derivat oder Salze davon nach Anspruch 1, wobei das Aryl ein unsubstituiertes Phenyl oder Naphthyl oder ein Phenyl ist, das durch Alkyl, Alkoxy, Hydroxy, Halogen, Carboxyl, Alkloxyacyl, N,N-Dialkylamino oder heterocycliches Alkylacyl substituiert ist; wobei N-Alkylamino n-Propylamino, n-Butylamino, Isopropylamino, Isobutylamino oder N-Hydroxy-ethylamino ist; und N,N-Dialkylamino die gleichen oder unterschiedliche Alkylsubstituenten aufweist und N,N-Dimethylamino, N,N-Diethylamino, N,N-Methylethylamino, N-Methyl-N-morpholino-ethylamino, N-Methyl-N-hydroxyethylamino oder N-Methyl-N-anilino umfasst.

3. Chirales 3-Hydroxypyridin-4-on-Derivat oder Salze davon nach Anspruch 1 oder 2, wobei die Salze durch eine Verbindung der Formel **I** und eine Säure gebildet sind, die aus starker anorganischer Säure, einer Alkylcarbonsäure, gesättigter oder ungesättigter Dicarbonsäure oder Methyl- oder p-Toluolsulfonsäure ausgewählt ist.

4. Chirales 3-Hydroxypyridin-4-on-Derivat oder Salze davon nach Anspruch 3, wobei die starke anorganische Säure eine Mineralsäure ist und Sulfonsäure, Phosphorsäure oder Chlorwasserstoffsäure umfasst; die Alkylcarbonsäure Essigsäure umfasst; die gesättigte oder ungesättigte Dicarbonsäure Weinsäure oder Zitronensäure umfasst.

5. Chirales 3-Hydroxypyridin-4-on-Derivat oder Salze davon nach einem der Ansprüche 1 bis 4, wobei:
(a) R₁ in Formel **I** Methyl ist und eine Verbindung in Formel **II** gezeigt ist: 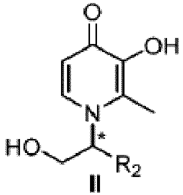 oder
(a) R₁ in Formel **I** Ethyl ist und eine Verbindung in Formel **III** gezeigt ist: 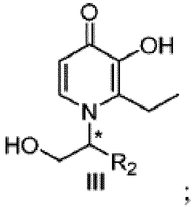
wobei das mit "*" markierte Kohlenstoffatom chiral ist und R₂ wie in Formel I definiert ist.

6. Chirales 3-Hydroxypyridin-4-on-Derivat oder Salze davon nach Anspruch 5, ausgewählt aus Verbindung III-c: (*R*)-3-Hydroxy-1-(1-hydroxy-3-phenylpropyl-2-)-2-ethyl pyridin-4(1*H*)-on oder Verbindung II-c: (*R*)-3-Hydroxy-1-(1-hydroxy-3-phenylpropyl-2-)-2-methylpyridin-4(1*H*)-on.

7. Verfahren zum Herstellen des chiralen 3-Hydroxypyridin-4-on-Derivats oder von Salzen davon nach einem der Ansprüche 1 bis 6, wobei das Verfahren die folgenden Schritte umfasst: Reagieren von Verbindung **V** mit Verbindung **VI,** wodurch Verbindung **VII** gebildet wird, die an Position 3 benzylgeschützt ist, danach Reagieren von Verbindung **VII** mit Verbindung **VIII,** wodurch Verbindung **IX** gebildet wird, und schließlich Entschützen von Verbindung **IX** in der Gegenwart eines Pd/C-Katalysators über eine Hydrierungsreduktion, wodurch eine Verbindung der Formel **I** gebildet wird, und wobei das Reaktionsschema wie folgt ist:

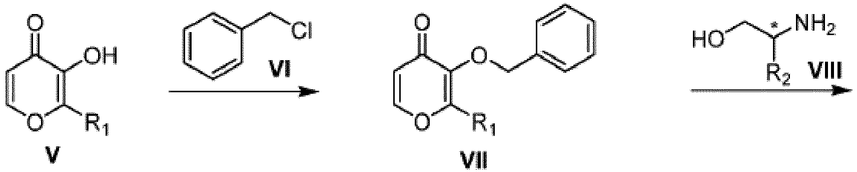

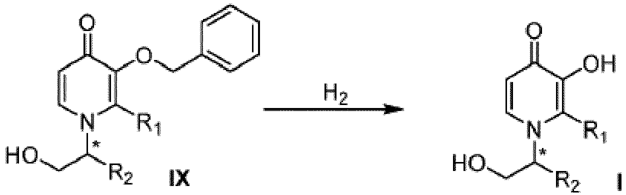

8. Verfahren nach Anspruch 7, das des Weiteren den Schritt des und Reagierens einer Verbindung der Formel **I** mit einer anorganischen oder organischen Säure HX umfasst, um entsprechende Salze zu bilden, und wobei das Reaktionsschema wie folgt ist:

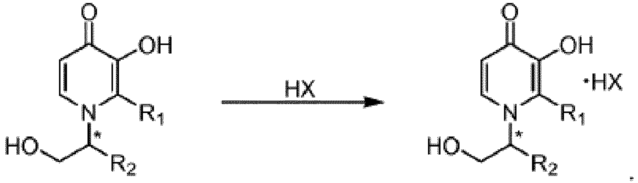

9. Chirales 3-Hydroxypyridin-4-on-Derivat oder Salze davon nach einem der Ansprüche 1 bis 6 zur Verwendung als Medikament zur Behandlung einer Krankheit, die aus der Gruppe ausgewählt ist, bestehend aus Thalassämie, eisenvermittelten Gewebeläsionen bei neurodegenerativen Krankheiten und onkologischen Erkrankungen.

10. Chirales 3-Hydroxypyridin-4-on-Derivat oder Salze davon zur Verwendung nach Anspruch 9, wobei das chirale 3-Hydroxypyridin-4-on-Derivat oder Salze davon nach einem der Ansprüche 1 bis 6 in eine Formulierung formuliert ist bzw. sind, die eine flüssige Zubereitung oder eine feste Zubereitung umfasst, und wobei die Verabreichungsmethode eine orale Verabreichung oder eine Verabreichung durch Injektion ist.

11. Medikament, das das chirale 3-Hydroxypyridin-4-on-Derivat oder Salze davon nach einem der Ansprüche 1 bis 6 umfasst.

12. Medikament nach Anspruch 11, wobei das Medikament das chirale 3-Hydroxypyridin-4-on-Derivat oder Salze davon als Wirkstoff und einen pharmazeutisch unbedenklichen Träger oder Hilfsstoff umfasst.

13. Medikament nach Anspruch 11 oder 12, wobei das Medikament in flüssiger Form oder fester Form formuliert ist.

14. Medikament nach Anspruch 11 bis 13 zur Verwendung bei der Behandlung einer Krankheit, die aus der Gruppe ausgewählt ist, bestehend aus Thalassämie, eisenvermittelten Gewebeläsionen bei neurodegenerativen Krankheiten und onkologischen Erkrankungen.

## Revendications

1. Dérivé de 3-hydroxypyridin-4-one chiral ou sels de celui-ci ayant la formule générale suivante **I** : 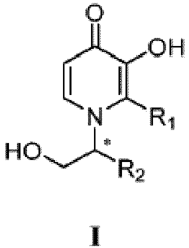 **caractérisé par le fait que** l'atome de carbone marqué par « * » est chiral,
R₂ représente aralkyle, ou aralkyle hétérocyclique ; où :
- l'aralkyle se réfère à une chaîne d'alkyle qui contient au moins un atome d'hydrogène, où un atome d'hydrogène attaché au carbone terminal est remplacé par un aryle, comprenant benzyle, p-chlorobenzyle, o-fluorobenzyle, phénéthyle, p-méthylbenzyle, p-diméthylamino benzyle, p-diéthylamino benzyle, p-cyanobenzyle ou p-pyrrolidinyl benzyle ;
- l'aralkyle hétérocyclique se réfère à une chaîne d'alkyle qui contient au moins un atome d'hydrogène, où un atome d'hydrogène attaché à l'atome de carbone terminal est remplacé par un hétérocycle aromatique, où l'hétérocycle aromatique a un ou plusieurs substituants choisis parmi alkyle, halogène, trifluorométhyle, carboxyle, alcoxyacyle, N-alkylamino, N,N-dialkylamino ou pyrrolidinyle ; et
- R₁ représente alkyle, hydroxy alkyle, mercapto alkyle, haloalkyle, alcoxy, alkylthiol, haloalcoxy, haloalkylthiol, carboxyl alkyle, alcoxyacyl-alkyle, R₃R₄N-C(O)-alkyle, hétérocycloalkyle, aryle non substitué, aroyle, aralkyle, aryle hétérocyclique non substitué ou aralkyle hétérocyclique, où :
(i) l'alkyle représente méthyle, éthyle, n-propyle, isopropyle, n-butyle, isobutyle, n-pentyle, n-hexyle ou n-heptyle ;
(ii) l'hydroxy alkyle est un alkyle substitué par hydroxy, où hydroxyle est à l'extrémité de l'alkyle ou au milieu de l'alkyle, et comprend 3-hydroxypropyle, 2-hydroxypropyle, 1-hydroxypropyle ou 1-hydroxy-2-méthylpropyle ;
(iii) le mercapto alkyle se réfère à un groupe dans lequel l'atome d'oxygène dans hydroxy alkyle est remplacé par un atome de soufre ;
(iv) l'halogène alkyle se réfère à un alkyl substitué par un atome d'halogène choisi parmi le chlore ou le fluor, ou substitué par trichloro ou trifluoro, et le site de substitution par l'halogène est à l'extrémité ou au milieu de l'alkyle ;
(v) le groupe alcoxy se réfère à un groupe formé par l'alkyle et un atome d'oxygène qui est lié de façon covalente au site de substitution par une liaison éther, et comprend méthoxy, oxéthyle, n-propoxy, isopropoxy, n-butoxy, isobutoxy, sec-butoxy, tert-butoxy, n-pentyloxy, isopentyloxy ;
(vi) le groupe alkylthiol est un groupe dans lequel l'atome d'oxygène dans alcoxy est remplacé par un atome de soufre ;
(vii) l'haloalcoxy se réfère à un alcoxy substitué par un atome d'halogène choisi parmi le chlore ou le fluor, ou substitué par trichloro ou trifluoro ;
(viii) l'haloalkylthiol se réfère à un groupe dans lequel l'atome d'oxygène dans haloalcoxy est remplacé par un atome de soufre ;
(ix) le carboxyl alkyle se réfère à un alkyl substitué par carboxyle comprenant une seule substitution par carboxyle, ou de multiples substitutions par carboxyle, et le site de substitution est à l'extrémité ou au milieu de l'alkyle ;
(x) l'alcoxyacyle se réfère à « alkyl-O-C(O)- », et comprend méthoxycarbonyle, éthoxycarbonyle, n-propoxycarbonyle, isopropoxycarbonyle, isobutoxycarbonyle, isobutoxycarbonyle, secbutoxycarbonyle, tert-butoxycarbonyle, n-pentyloxycarbonyle ou isopentyloxycarbonyle ;
(xi) R₃ et R₄ dans R₃R₄N-C(O)-alkyle sont identiques ou différents et représentent hydrogène, alkyle, hydroxy alkyle, alcoxy-alkyle, hydroxy alcoxy-alkyle, amino-alkyle, N-alkylamino-alkyle, N,N-dialkylamino-alkyle, N-hydroxyalkylamino-alkyle, N,N-dihydroxyalkylamino-alkyle, ou un noyau alicyclique contenant de l'azote formé par liaison de deux extrémités d'alkyle avec un atome d'azote ;
(xii) l'alkyle hétérocyclique se réfère à une structure de noyau contenant 3-8 atomes, où il y a au moins un hétéroatome choisi parmi un atome d'oxygène, d'azote ou de soufre ;
(xiii) l'aryle non substitué se réfère à un phényle non substitué ;
(xiv) l'aroyle se réfère à « aryl-C(O)- », et comprend benzoyle, toluoyle, naphtoyle ou p-méthoxy benzoyle ;
(xv) l'aralkyle se réfère à une chaîne d'alkyle qui contient au moins un atome d'hydrogène, où un atome d'hydrogène attaché au carbone terminal est remplacé par un aryle, et comprend benzyle, *p-*chlorobenzyle, *o*-fluorobenzyle, phénéthyle, *p-*méthylbenzyle, *p*-diméthylamino benzyle, *p-*diéthylamino benzyle, *p*-cyanobenzyle ou *p-*pyrrolidinyl benzyle ;
(xvi) l'aryle hétérocyclique se réfère à un noyau aromatique contenant un hétéroatome, et comprend hétérocycle aromatique-alkyle ; l'hétérocycle aromatique se réfère à un noyau aromatique contenant 3-7 atomes où il y a au moins un hétéroatome, et comprend pyrrolidinyle, imidazolyle, triazolyle, tétrazolyle, oxygène-azote cyclopentadiényle, thiazolyle, furanyle, thiophényle, pyridyle, pyrazinyle, thiazinyle, pyranyle ou pyrimidyle ; l'hétérocycle aromatique est non substitué ;
(xvii) l'aralkyle hétérocyclique se réfère à une chaîne d'alkyle qui contient au moins un atome d'hydrogène, où un atome d'hydrogène attaché à l'atome de carbone terminal est remplacé par un hétérocycle aromatique ; et l'hétérocycle aromatique a un ou plusieurs substituants choisis parmi alkyle, halogène, trifluorométhyle, carboxyle, alcoxyacyle, N-alkylamino, N,N-dialkylamino ou pyrrolidinyle.

2. Dérivé de 3-hydroxypyridin-4-one chiral ou sels de celui-ci selon la revendication 1, dans lequel l'aryle est un phényle ou naphtyle non substitué, ou un phényle substitué par alkyle, alcoxy, hydroxy, halogène, carboxyle, alcoxyacyle, N,N-dialkylamino ou alkylacyle hétérocyclique ; où N-alkylamino est n-propylamino, n-butylamino, isopropylamino, isobutylamino ou N-hydroxy-éthylamino ; et N,N-dialkylamino a des substituants alkyle identiques ou différents et comprend N,N-diméthylamino, N,N-diéthylamino, N,N-méthyléthylamino, N-méthyl-N-morpholino-éthylamino, N-méthyl-N-hydroxyéthylamino ou N-méthyl-N-anilino.

3. Dérivé de 3-hydroxypyridin-4-one chiral ou sels de celui-ci selon l'une des revendications 1 ou 2, dans lequel les sels sont formés par un composé de formule **I** et un acide choisi parmi un acide inorganique fort, un acide alkyl carboxylique, un acide dicarboxylique saturé ou insaturé ou un acide méthyl ou p-toluènesulfonique.

4. Dérivé de 3-hydroxypyridin-4-one chiral ou sels de celui-ci selon la revendication 3, dans lequel l'acide inorganique fort est un acide minéral, et comprend l'acide sulfonique, l'acide phosphorique ou l'acide chlorhydrique ; l'acide alkyl carboxylique comprend l'acide acétique ; l'acide dicarboxylique saturé ou insaturé comprend l'acide tartrique ou l'acide citrique.

5. Dérivé de 3-hydroxypyridin-4-one chiral ou sels de celui-ci selon l'une quelconque des revendications 1 à 4, dans lequel :
(a) R₁ dans la formule **I** représente méthyle, et le composé est représenté dans la formule **II** : 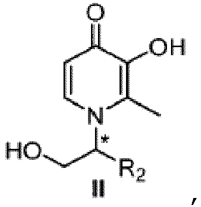 ou
(b) R₁ dans la formule **I** représente éthyle, et le composé est représenté dans la formule **III** : 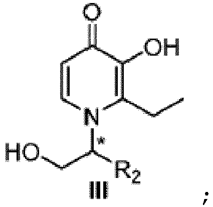 où l'atome de carbone marqué par « * » est chiral et R₂ est tel que défini dans la formule **I**.

6. Dérivé de 3-hydroxypyridin-4-one chiral ou sels de celui-ci selon la revendication 5 choisi parmi le Composé III-c: (*R*)-3-hydroxy-1-(1-hydroxy-3-phénylpropyl-2-)-2-éthyl pyridine-4(1*H*)-one ou le Composé II-c : (*R*)-3-hydroxy-1-(1-hydroxy-3-phényl propyl-2-)-2-méthyl pyridine-4(1*H*)-one.

7. Procédé de préparation du dérivé de 3-hydroxypyridin-4-one chiral ou de sels de celui-ci selon l'une quelconque des revendications 1 à 6, dans lequel ledit procédé comprend les étapes suivantes : faire réagir le composé **V** avec le composé **VI,** formant par là le composé **VII** qui est protégé par benzyle à la position 3, puis faire réagir le composé **VII** avec le composé **VIII,** formant par là le composé **IX**, et finalement, en présence de catalyseur Pd/C, déprotéger le composé **IX** par une réduction d'hydrogénation, formant par là le composé de formule **I**, et le schéma réactionnel est comme suit :

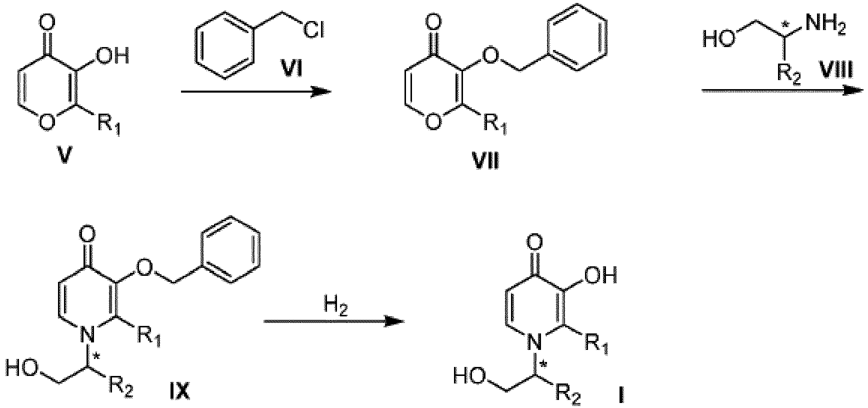

8. Procédé selon la revendication 7 comprenant en outre l'étape de réaction du composé de formule **I** avec un acide inorganique ou organique HX pour former les sels correspondants, et le schéma réactionnel est comme suit :

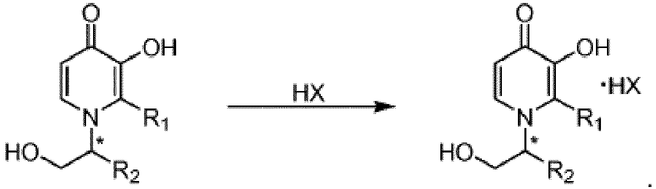

9. Dérivé de 3-hydroxypyridin-4-one chiral ou sels de celui-ci selon l'une quelconque des revendications 1 à 6 pour une utilisation comme médicament pour le traitement d'une maladie choisie dans le groupe consistant en thalassémie, lésions tissulaires médiées par le fer dans les maladies neurodégénératives et les maladies oncologiques.

10. Dérivé de 3-hydroxypyridin-4-one chiral ou sels de celui-ci pour une utilisation selon la revendication 9, dans lequel le dérivé de 3-hydroxypyridin-4-one chiral ou les sels de celui-ci selon l'une quelconque des revendications 1 à 6 est formulé en une formulation comprenant une préparation liquide ou une préparation solide, et le mode d'administration est l'administration orale ou l'administration par injection.

11. Médicament comprenant le dérivé de 3-hydroxypyridin-4-one chiral ou des sels de celui-ci selon l'une quelconque des revendications 1 à 6.

12. Médicament selon la revendication 11, dans lequel le médicament comprend le dérivé de 3-hydroxypyridin-4-one chiral ou des sels de celui-ci comme principe actif et un support ou excipient pharmaceutiquement acceptable.

13. Médicament selon l'une des revendications 11 ou 12, dans lequel le médicament est formulé sous forme liquide ou sous forme solide.

14. Médicament selon les revendications 11 à 13, pour une utilisation dans le traitement d'une maladie choisie dans le groupe consistant en thalassémie, lésions tissulaires médiées par le fer dans les maladies neurodégénératives et les maladies oncologiques.
